# EUROPEAN PATENT SPECIFICATION

(11) **EP 1 225 227 B1**
(45) Date of publication and mention of the grant of the patent: **18.02.2009**
(21) Application number: 02005972.1
(22) Date of filing: 10.12.1991
(51) Int. Cl.: C12N 15/56, C12N 9/24, C12N 15/80, C12N 15/90, C12Q 1/68, C12P 19/14, C11D 3/386

(54) **Improved saccharification of cellulose by cloning and amplification of the beta-glucosidase gene of trichoderma reesei**
Verbesserte Saccharifizierung von Zellulose durch Klonierung und Vervielfältigung des beta-Glukosidase Genes aus Trichoderma
Saccharification améliorée de la cellulose par clonage et amplification du gène de la beta-glucosidase de trichoderma reesei

(30) Priority: 10.12.1990 US 625140
(43) Date of publication of application: 24.07.2002
(62) Divisional of application: 92902669.8
(73) Proprietor: GENENCOR INTERNATIONAL, INC., Palo Alto, California 94304 (US)
(72) Inventor: Fowler, Timothy, Belmont, CA 94002 (US); Barnett, Christopher C., South San Francisco, CA 94080 (US); Shoemaker, Sharon, Fairfield, CA 94533 (US)
(74) Representative: Kremer, Simon Mark

(56) References cited:
- EP-A- 0 121 397
- EP-A- 0 244 234
- WO-A-91/17244
- WO-A-91/18090
- WO-A-92/06209
- WO-A-92/06210
- FR-A- 2 615 527
- US-A- 4 275 163
- US-A- 4 745 062
- STRAUSS J ET AL: "BETA GLUCOSIDASE AND CELLULASE FORMATION BY A TRICHODERMA-REESEI MUTANT DEFECTIVE IN CONSTITUTIVE BETA GLUCOSIDASE FORMATION" JOURNAL OF GENERAL MICROBIOLOGY, vol. 136, no. 7, 1990, pages 1321-1326, XP008003898 ISSN: 0022-1287
- MISHRA S ET AL: "ISOLATION AND CHARACTERIZATION OF A MUTANT OF TRICHODERMA-REESEI SHOWING REDUCED LEVELS OF EXTRACELLULAR BETA GLUCOSIDASE" JOURNAL OF GENERAL MICROBIOLOGY, vol. 135, no. 12, 1989, pages 3459-3466, XP008003946 ISSN: 0022-1287
- GRUBER F ET AL: "CLONING OF THE TRICHODERMA-REESEI PYRG GENE AND ITS USE AS A HOMOLOGOUS MARKER FOR A HIGH-FREQUENCY TRANSFORMING SYSTEM" CURRENT GENETICS, vol. 18, no. 5, 1990, pages 447-451, XP008003896 ISSN: 0172-8083
- CHENG C ET AL: "TRANSFORMATION OF TRICHODERMA-VIRIDE USING THE NEUROSPORA-CRASSA PYR4 GENE AND ITS USE IN THE EXPRESSION OF A TAKA-AMYLASE A GENE FROM ASPERGILLUS-ORYZAE" CURRENT GENETICS, vol. 18, no. 5, 1990, pages 453-456, XP008003897 ISSN: 0172-8083
- ALANI E ET AL: "A method for gene disruption that allows repeated use of URA3 selection in the construction of multiply disrupted yeast strains" GENETICS, GENETICS SOCIETY OF AMERICA, AUSTIN, TX, US, vol. 116, August 1987 (1987-08), pages 541-545, XP002108451 ISSN: 0016-6731
- DATABASE WPI Section Ch, Week 198843 Derwent Publications Ltd., London, GB; Class D17, AN 1988-305166 XP002200638 & JP 63 226294 A (AJINOMOTO KK), 20 September 1988 (1988-09-20)

## Description

### BACKGROUND OF THE INVENTION

### 1. Field of the Invention.

The present invention relates to cellulase preparations and compositions having increased or decreased cellulolytic capacity. The invention further relates to a nucleotide sequence of the bgl1 gene encoding extracellular β-glucosidase from a filamentous fungi, a plasmid vector containing the gene encoding extracellular β-glucosidase from which the coding region for the gene has been removed. More particularly, the present invention relates to Trichoderma reesei strains that have increased or no levels of expression of the bgl1 gene resulting in enhanced or no extracellular β-glucosidase protein levels that can be used in conjunction with other compositions to produce a cellulase product having increased or decreased cellulolytic capacity.

### 2. State of the Art.

Cellulases are known in the art as enzymes that hydrolyze cellulose (β-1,4-glucan linkages), thereby resulting in the formation of glucose, cellobiose, cellooligosaccharides, and the like. As noted by Wood et al., "Methods in Enzymology", 160, 25, pages 234 et seq. (1988) and elsewhere, cellulase produced by a given microorganism is comprised of several different enzyme classes including those identified as exocellobiohydrolases (EC 3.2.1.91) ("CBH"), endoglucanases (EC 3.2.1.4) ("EG"), β-glucosidases (EC 3.2.1.21) ("BG"). Moreover, the fungal classifications of CBH, EG and BG can be further expanded to include multiple components within each classification. For example, multiple CBHs and EGs have been isolated from a variety of bacterial and fungal sources including Trichoderma reesei which contains 2 CBHs, i.e., CBH I and CBH II, and at least 3 EGs, i.e., EG I, EG II, and EG III components.

The complete cellulase system comprising components from each of the CBH, EG, and BG classifications is required to efficiently convert crystalline forms of cellulose to glucose. Isolated components are far less effective, if at all, in hydrolyzing crystalline cellulose. Moreover, a synergistic relationship is observed between the cellulase components particularly if they are of different classifications. That is to say, the effectiveness of the complete cellulase system is significantly greater than the sum of the contributions from the isolated components of the same classification. In this regard, it is known in the art that the EG components and CBH components synergistically interact to more efficiently degrade cellulose. See, for example, Wood, Biochem. Soc. Trans., 13, pp. 407-410 (1985).

The substrate specificity and mode of action of the different cellulase components varies with classification, which may account for the synergy of the combined components. For example, the current accepted mode of cellulase action is that endoglucanase components hydrolyze internal β-1,4-glucosidic bonds, particularly, in regions of low crystallinity of the cellulose and exo-cellobiohydrolase components hydrolyze cellobiose from the non-reducing end of cellulose. The action of endoglucanase components greatly facilitates the action of exo-cellobiohydrolases by creating new chain ends which are recognized by exo-cellobiohydrolase components.

β-Glucosidases are essential components of the cellulase system and are important in the complete enzymatic breakdown of cellulose to glucose. The β-glucosidase enzymes can catalyze the hydrolysis of alkyl and/or aryl β-D-glucosides such as methyl β-D-glucoside and p-nitrophenyl glucoside, as well as glycosides containing only carbohydrate residues, such as cellobiose. The catalysis of cellobiose by β-glucosidase is important because it produces glucose for the microorganism and further because the accumulation of cellobiose inhibits cellobiohydrolases and endoglucanases thus reducing the rate of hydrolysis of cellulose to glucose.

Since β-glucosidases can catalyze the hydrolysis of a number of different substrates, the use of this enzyme in a variety of different applications is possible. For instance, some β-glucosidases can be used to liberate aroma in fruit by catalyzing various glucosides present therein. Similarly, some β-glucosidases can hydrolyze grape monoterpenyl β-glucosidase which upon hydrolysis, represents an important potential source of aroma to wine as described by Günata et al, "Hydrolysis of Grape Monoterpenyl β-D-Glucosides by Various β-Glucosidases", J. Agric. Food Chem., Vol. 38, pp. 1232-1236 (1990).

Furthermore, cellulases can be used in conjunction with yeasts to degrade biomass to ethanol wherein the cellulose degrades cellobiose to glucose that yeasts can further ferment into ethanol. This production of ethanol from readily available sources of cellulose can provide a stable, renewable fuel source. The use of ethanol as a fuel has many advantages compared to petroleum fuel products such as a reduction in urban air pollution, smog, and ozone levels, thus enhancing the environment. Moreover, ethanol as a fuel source would reduce the reliance on foreign oil imports and petrochemical supplies.

But the major rate limiting step to ethanol production from biomass is the insufficient amount of β-glucosidase in the system to efficiently convert cellobiose to glucose. Therefore, a cellulase composition that contains an enhanced amount of β-glucosidase would be useful in ethanol production.

Contrarily, in some cases, it is desirable to produce a cellulase composition which is deficient in, and preferably free of β-glucosidase. Such compositions would be advantageous in the production of cellobiose and other cellooligosaccharides.

β-glucosidases are present in a variety of prokaryotic organisms, as well as eukaryotic organisms. The gene encoding β-glucosidase has been cloned from several prokaryotic organisms and the gene is able to direct the synthesis of detectable amounts of protein in E. coli without requiring extensive genetic engineering, although, in some cases, coupling with a promotor provided by the vector is required. However, β-glucosidases are not produced by such organisms in commercially feasible amounts.

Furthermore, such prokaryotic genes often cannot be expressed and detected after transformation of the eukaryotic host. Thus, in order to use fungal strains, fungal genes would have to be cloned using methods described herein or by detection with the T. reesei bgl1 gene by nucleic acid hybridization.

The contribution and biochemistry of the β-glucosidase component in cellulose hydrolysis is complicated by the apparent multiplicity of enzyme forms associated with T. reesei and other fungal sources (Enari et al, "Purification of Trichoderma reesei and Aspergillus niger β-glucosidase", J. Appl. Biochem., Vol. 3, pp. 157-163 (1981); Umile et al, "A constitutive, plasma membrane bound β-glucosidase in Trichoderma reesei", FEMS Microbiology Letters, Vol. 34, pp. 291-295 (1986); Jackson et al, "Purification and partial characterization of an extracellular β-glucosidase of Trichoderma reesei using cathodic run, polyacrylamide gel electrophoresis", Biotechnol. Bioeng., Vol. 32, pp. 903-909 (1988)). These and many other authors report β-glucosidase enzymes ranging in size from 70-80 Kd and in pI from 7.5-8.5. More recent data suggests that the extracellular and cell wall associated forms of β-glucosidase are the same enzyme (Hofer et al, "A monoclonal antibody against the alkaline extracellular β-glucosidase from Trichoderma reesei: reactivity with other Trichoderma β-glucosidases", Biochim. Biophys. Acta, Vol. 992, pp. 298-306 (1989); Messner and Kubicek, "Evidence for a single, specific β-glucosidase in cell walls from Trichoderma reesei QM9414", Enzyme Microb. Technol., Vol. 12, pp. 685-690 (1990)) and that the variation in size and pI is a result of post translational modification and heterogeneous methods of enzyme purification. It is unknown whether the intracellular β-glucosidase species with a pI of 4.4 and an apparent molecular weight of 98,000 is a novel β-glucosidase (Inglin et al, "Partial purification and characterization of a new intracellular β-glucosidase of Trichoderma reesei", Biochem. J., Vol. 185, pp. 515-519 (1980)) or a proteolytic fragment of the alkaline extracellular β-glucosidase associated with another protein (Hofer et al, supra).

Since a major part of the detectable β-glucosidase activity remains bound to the cell wall (Kubicek, "Release of carboxymethylcellulase and β-glucosidase from cell walls of Trichoderma reesei", Eur. J. Appl. Biotechnol., Vol. 13, pp. 226-231 (1981); Messner and Kubicek, supra; Messner et al, "Isolation of a β-glucosidase binding and activating polysaccharide from cell walls of Trichoderma reesei", Arch. Microbiol., Vol. 154, pp. 150-155 (1990)), commercial preparations of cellulase are thought to be reduced in their ability to produce glucose because of relatively low concentrations of β-glucosidase in the purified cellulase preparation.

To overcome the problem of β-glucosidase being rate limiting in the production of glucose from cellulose using cellulase produced by a filamentous fungi, the art discloses supplementation of the cellulolytic system of Trichoderma reesei with the β-glucosidase of Aspergillus and the results indicate an increase in rate of saccharification of cellulose to glucose. Duff, Biotechnol Letters, 7, 185 (1985). Culturing conditions of the fungi have also been altered to increase β-glucosidase activity in Trichoderma reesei as illustrated in Sternberg et al, Can. J. Microbiol., 23, 139 (1977) and Tangnu et al, Biotechnol. Bioeng., 23, 1837 (1981), and mutant strains obtained by ultraviolet mutation have been reported to enhance the production of β-glucosidase in Trichoderma reesei. Although these aforementioned methods increase the amount of β-glucosidase in Trichoderma reesei, the methods lack practicality and, in many instances, are not commercially feasible.

Strauss et al (Journal of General Microbiology, vol. 136, no. 7, 1990, pages 1321-1326) disclose a mutant of *Trichoderma reesei* isolated after γ-irradiation, which did not form β-glucosidase during growth on glycerol or glucose, but secreted β-glucosidase upon growth on cellobiose. The mutant produced cellulases upon growth on lactose and after a lag on cellulose.

Mishra et al (Journal of General Microbiology, vol. 135, no. 12, 1989, pages 3549-3465) discuss the physiological and biochemical characterisation of a *T. reesei* mutant that produced low levels of extracellular β-glucosidase.

EP 0 244 234 discloses the development of a vector system for transformation of *Trichoderma* which can be used for high expression and secretion of proteins in *Trichoderma* by transformation of a suitable *Trichoderma* strain with the vector system comprising a gene for a desired protein.

FR 2 615 527 relates to a new yeast strain, obtained by integration of an integrative vector containing a gene encoding a specific protein, especially the gene MEL1, at the locus of a cryptic gene, especially the gene for β-glucosidase, of an ascosporogenic yeast.

Gruber et al (Current Genetics, 1990, vol. 18, pages 447-451) discusses the isolation of the *T. reesei* orotidine-5'-phosphate decarboxylase gene, by heterologous hybridization with the corresponding *Neurospora* gene as a probe. A 2.7 kb *Sal*I fragment, which exclusively hybridized to the *Neurospora* gene was subcloned in pGEM-5Zf (+) and this subclone was used to transform a *T*. *reesei pyrG⁻* negative mutant to PYR⁺.

Cheng et al (Current Genetics, 1990, vol. 18, pages 453-456) relates to the isolation of a *pyrG* mutant of *Trichoderma viride*, transformation of *Trichoderma viride* using the *Neurospora crassa pyr*4 gene and its use in the expression of a Taka-amylase A gene from *Aspergillus oryzae.*

Alani et al (Genetics, Genetics Society of America, 1987, vol. 116, pages 541-545) describe a 3.8kb molecular construct used to disrupt yeast genes, consisting of a functional yeast *URA*3 gene flanked by 1.1kb direct repeats of a bacterial sequence.

US 4,745,062 relates to a plasmid vector for cloning and expression of a protein in a microorganism which comprises at least one structural gene which codes the synthesis of the said protein and elements which ensure the expression of the said structural gene in a microorganism, and wherein the promotion of the structural gene is ensured by the expression promotor of the beta -glucosidase gene in yeasts. It also describes microorganisms transformed by the said vectors, in particular a transformed strain of *S. cerevisiae*, a fermentation process using the vectors, and the enzymes prepared by said process, in particular β-glucosidase.

EP 0 121 397 discloses a process for producing enzymes capable of catalyzing the hydrolysis of cellulose comprising the steps of inoculating a growth medium containing an inducer of cellulose synthesis with *T*. *reesei* strain MCG 80 and incubating the inoculated growth medium under conditions which will permit the growth of MCG 80.

US 4,275,163 describes a process and a microorganism for synthesizing cellulase enzymes. The microorganism is a mutant strain of an *Ascomycete* fungus capable of synthesizing cellulases. The synthesis of cellulases by the mutant is not repressed by glycerol, repressed by glucose but not subject to post-repression lag, and inducible to high levels by lactose.

JP 63 226294 relates to a method for the production of cellubiose involving the use of a cellulase- containing enzyme agent, wherein the cellulase is produced by microorganisms of *Sporotrichum* genus, *Trichoderma* genus, *Aspergillus* genus, *Irpex* genus, etc.

WO 91/17244 discloses an enzyme capable of degrading cellulose or hemicellulose derivable from a fungus other than *Trichoderma* or *Phanerochaete* which comprises a carbohydrate binding domain homologous to a terminal A region of *Trichoderma reesei* cellulases.

WO 91/18090 relates to enzyme preparations enriched in hemicellulase-, pectin-, and/or lignin-degrading enzymes are described which are also partially or completely deficient in cellulase degrading activity. Methods for the production of such enzyme preparations by genetically engineering members of the species *Trichoderma* are disclosed.

In order to transform mutant strains of Trichoderma reesei or other filamentous fungi, the amino acid sequence of the bgll gene of Trichoderma reesei or the other filamentous fungi must be first characterize so that the bgll gene can be cloned and introduced into mutant strains of Trichoderma reesei or other filamentous fungi.

Additionally, once the bgl1 gene has been identified, information within linear fragments of the bgl1 gene can be used to prepare strains of Trichoderma reesei and other filamentous fungi which produce cellulase compositions free of β-glucosidase.

Accordingly, this invention is directed, in part, to the characterization of the bgl1 gene that encodes for extracellular or cell wall bound β-glucosidase from Trichoderma reesei and other filamentous fungi. This invention is further directed to the cloning of the bgl1 gene into a plasmid vector that can be used in the transformation process.

This invention is further directed, in part, to the deletion or disruption of the bgl1 gene from the Trichoderma reesei or other filamentous fungi genome.

### SUMMARY OF THE INVENTION

The amino acid sequence of the extracellular or cell wall bound β-glucosidase protein from Trichoderma reesei has now been obtained in sufficient detail to enable the bgl1 gene to be cloned into a suitable plasmid vector.

Accordingly, in one of its process aspects.

the present invention relates to a process for expressing cellulases from a β-glucosidic filamentous fungi which are free of extracellular β-glucosidase, the process being as set out in the claims.

In one of its composition aspects, the present invention is directed to novel and useful transformants of Trichoderma reesei, which can be used to produce fungal cellulase compositions, especially fungal cellulase compositions deleted of β-glucosidase.

### BRIEF DESCRIPTION OF THE DRAWINGS

Fig. 1 is the nucleotide sequence and deduced primary amino acid structure of the entire T. reesei bgl1 gene.
Fig. 2 is a schematic representation of the vector pSASβ-glu.
Fig. 3A is a figurative representation of the vector pSASΔβGlu bal pyr (Δ36).
Fig. 3B is a figurative representation of the vector pUCΔβ-Glu A/R pyr (Δ12).
Fig. 4 represents a Northern blot of total RNA isolated from the transformed strains of Trichoderma reesei following induction with sophorose using the probes of cbh2 and a 700 bp fragment of bgl1 cDNA.
Fig. 5A represents an autoradiograph of a Southern blot of T. reesei DNA illustrating the presence of β-glucosidase gene in wild type T. reesei (RL-P37) compared to strains of T. reesei genetically modified so as to not include the β-glucosidase gene (Δ12 and Δ36).
Fig. 5B represents an autoradiograph of a Northern blot of T. reesei RNA illustrating the expression of β-glucosidase gene in wild type T. reesei (RL-P37) compared to strains of T. reesei genetically modified so as to not include the β-glucosidase gene (Δ12 and Δ36).
Fig. 5C represents an analysis of the proteins expressed by P37 (wild type), Δ12, and Δ36 strains of Trichorderma reesei and illustrates the absence of β-glucosidase in the proteins expressed by Δ12 and Δ36 strains Trichoderma reesei.
Fig. 6 represents an autoradiograph of Hind III digested genomic DNA from a T. reesei overproducing strain (lane 9) and transformants of pSASß-Glu (lanes 1-8), blotted and probed with the 700 bp β-Glu probe.

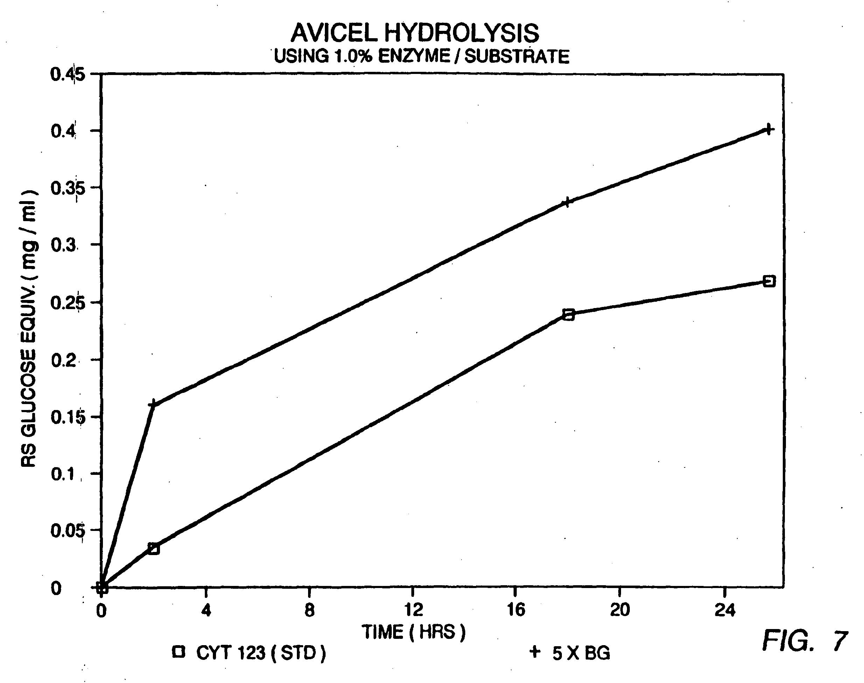

Fig. 7 represents a curve illustrating Avicel hydrolysis using the dosage, substrate:enzyme of 80:1 from an enriched recombinant β-glucosidase composition produced by the present invention.

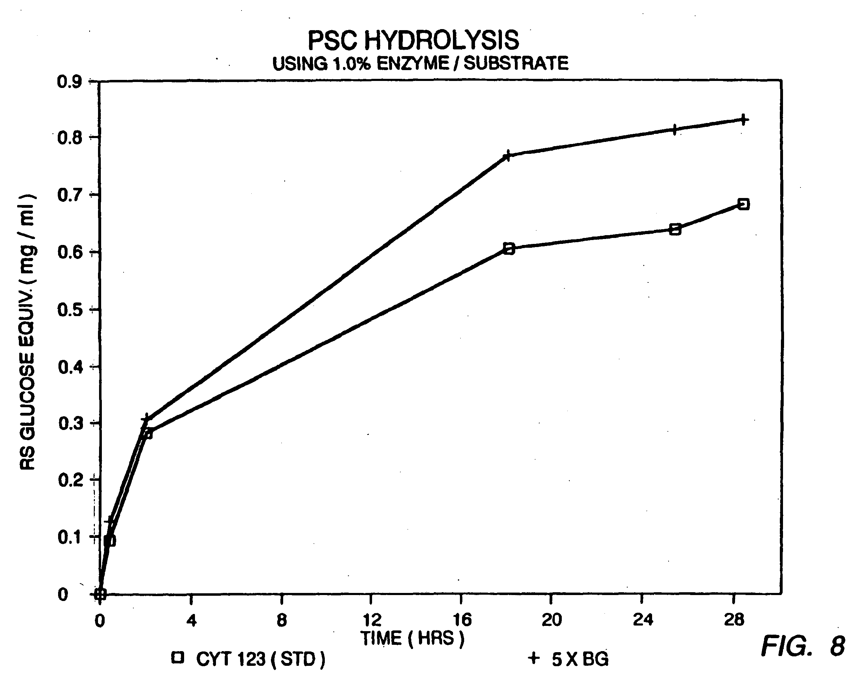

Fig. 8 represents a curve illustrating PSC hydrolysis using the dosage, substrate:enzyme of 300:1 from an enriched recombinant β-glucosidase composition produced by the present invention.

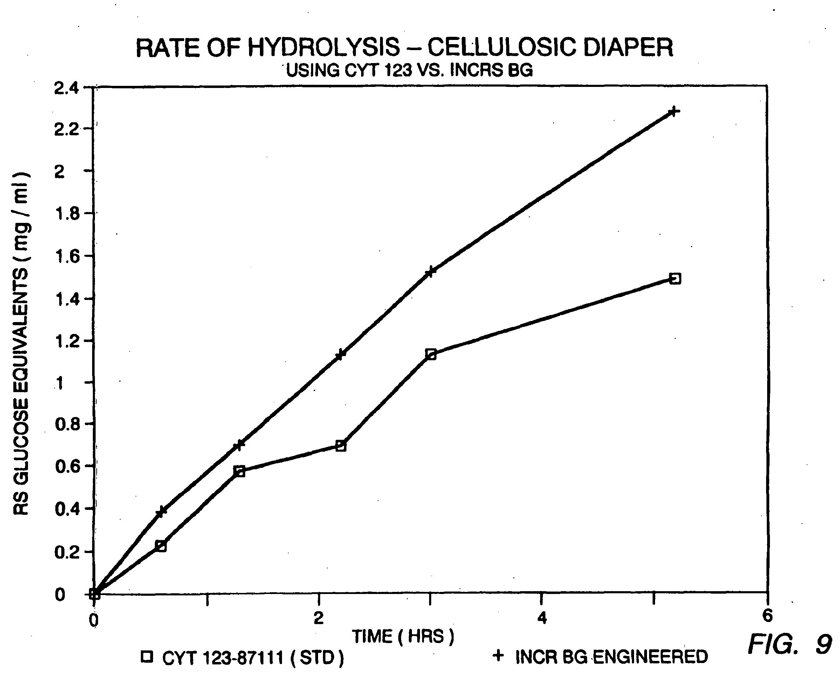

Fig. 9 represents a curve illustrating the rate of hydrolysis of a cellulosic diaper derived fibers using an enriched recombinant β-glucosidase composition produced by the present invention.
Figs. 10A and 10B are autoradiographs of Aspergillus nidulans, Neurospora crassa, Humicola grisea genomic DNA digested with Hind III and Eco RI, blotted and probed with a DNA fragment containing the bgl1 gene of Trichoderma reesei.

### DETAILED DESCRIPTION OF THE PREFERRED EMBODIMENTS OF THE INVENTION

The term "devoid of the bgl1 gene" means either that the bgl1 gene has been deleted from the genome and therefore cannot be expressed by the recombinant host microorganism; or that the bgl1 gene has been disrupted in the genome so that a functional extracellular β-glucosidase enzyme cannot be produced by the recombinant host microorganism.

The term "by recombinant means" denotes that a microorganism has been transformed with a DNA molecule created in a test-tube by ligating together pieces of DNA that are not normally contiguous.

The term "cellulase free of extracellular β-glucosidase" refers to a cellulase composition which does not contain functional extracellular β-glucosidase enzyme. Such compositions are preferably prepared by culturing a filamentous fungi wherein the β-glucosidase gene has been either deleted or disrupted. Preferably, these compositions are prepared by culturing a filamentous fungi wherein the β-glucosidase gene has been deleted.

The term "filamentous fungi" means any and all art recognized filamentous fungi.

The term "β-glucosidic filamentous fungi" refers to those filamentous fungi which produce a cellulase composition containing β-glucosidase.

The term "cellooligosaccharide" refers to those oligosaccharide groups containing from 2-8 glucose units having β-1,4 linkages. Such cellooligosaccharides include cellobiose (diglucose having a β-1,4- linkage) and are preferably derived from cellulose.

More specifically, the present invention discloses the isolation and characterization of the bgl1 gene coding for the extracellular or cell wall bound protein from Trichoderma reesei (sometimes referred to as "T. reesei") and the specific nucleotide and amino acid sequence of this gene. The bgl1 gene can be cloned into plasmid vectors, which are further used to produce transformed strains of T. reesei and other filamentous fungi having extra copies of the bgl1 gene inserted therein. These transformants are then used to produce cellulase compositions having increased β-glucosidase activity and thus enhanced cellulolytic degradation.

Besides enhancing cellulolytic degradation by inserting extra copies of the bgl1 gene into T. reesei strains, it is also contemplated by the present invention to produce transformed strains that are completely devoid of the bgl1 gene.

Generally, the present invention involves the isolation of the bgl1 gene from T. reesei by identifying a 700 bp cDNA fragment of the gene which is then used as a probe to identify a single T. reesei fragment containing the bgl1 gene which was subsequently cloned. Because of the species homology of the bgl1 gene, a probe employing a fragment of the bgl1 gene of T. reesei can be employed to identify the bgl1 gene in other cellulolytic microorganisms and, it is understood that the following description for T. reesei could also be applied to other β-glusosidic filamentous fungi.

In the case of T. reesei, this 6.0 kb fragment is then cloned into a pUC plasmid and a series of mapping experiments are performed to confirm that the entire bgl1 gene is contained in this fragment. The nucleotide sequence is then determined on both strands and the position of two introns can be confirmed by sequence analysis of bgl1 cDNA subclones spanning the intron/exon boundaries. After isolation of the bgl1 gene,

the entire bgl1 gene can also be deleted from the genome of T. reesei and other β-glucosidic filamentous fungi, thereby producing transformants that express cellulases free of β-glucosidase.

The isolation of the bgl1 gene from T. reesei involves the purification of extracellular β-glucosidase, chemical and proteolytic degradation of this protein, isolation and determination of the sequence of the proteolytic fragments and design of synthetic oligomer DNA probes using the protein sequence. The oligomeric probes are then further used to identify a 700 bp β-glucosidase cDNA fragment which can be labeled and employed to later identify a fragment that contains the entire bgl1 gene within the fragment from digested genomic DNA from T. reesei.

To identify a feasible cDNA fragment that can be used as a probe for future analysis, total RNA is first isolated from T. reesei mycelia and polyadenylated RNA isolated therefrom. The polyadenylated RNA is then used to produce a cDNA pool which is then amplified using specific oligonucleotide primers that amplify only the specific cDNA fragment encoding the T. reesei bgl1 gene.

More specifically, total RNA is first isolated from a starting strain of T. reesei. The starting strain employed in the present invention can be any T. reesei cellulase overproduction strain that is known in the art. This cellulase producing strain is generally developed by ordinary mutagenesis and selection methods known in the art from any T. reesei strain. Confirmation that the selected strain overproduces cellulases can be performed by using known analysis methods. A preferred strain is RLP37 which is readily accessible.

A mycelial inoculum from the T. reesei over production strain, grown in an appropriate growth medium, is added to a basal medium and incubated for a period of between 50-65 hours at a temperature between 25°C to 32°C, preferably 30°C. Fresh basal medium can be replaced during this incubation period. The culture medium is then centrifuged, and the mycelia is isolated therefrom and washed. The mycelia is then resuspended in a buffer to permit growth thereof and 1 mM sophorose (a β,1-2 dimer of glucose) is added to the mycelia to induce the production of cellulase enzymes. The mycelia preparation is then incubated for an additional time period, preferably 18 hours at 30°C prior to harvesting.

Total RNA can be isolated from the mycelia preparation by a variety of methods known in the art, such as proteinase K lysis, followed by phenol:chloroform extraction, guanidinium isothiocyanate extraction, followed by cesium chloride gradients, guanidine hydrochloride and organic solvent extraction, and the like. It is preferable to isolate total RNA via the procedure described by Timberlake et al in "Organization of a Gene Cluster Expressed Specifically in the Asexual Spores of A. nidulans," Cell, 26, pp. 29-37 (1981). The mycelia is isolated from the culture medium via filtration. Then the RNA is extracted from the mycelia by the addition of an extraction buffer, TE-saturated phenol and chloroform. The aqueous phase is removed and the organic phase is reextracted with the extraction buffer alone by heating the extraction mixture in a water bath at a temperature between about 60°C to 80°C, preferably 68°C to release the RNA trapped in polysomes and at the interface. All of the extracted aqueous phases are then pooled, centrifuged and reextracted with phenol-chloroform until there is no longer any protein at the interface. The RNA is further precipitated with 0.1 volume of 3 M sodium acetate and 2 volumes of 95% ethanol and pelleted via centrifugation before it is resuspended in DEP-water containing an RNase inhibitor.

The total RNA is then fractionated on 1% formaldehyde-agarose gels, blotted to Nytran™ membranes, and probed using a fragment of the T. reesei cbh2 gene to determine whether the genes encoding the enzymes of the cellulase system in the T. reesei preparation are indeed induced by addition of the sophorose. Basically, the probe used in the present invention is derived from a CBH II clone produced by methods known in the art. For more specific detail of how the clone was produced see Chen et al, "Nucleotide Sequence and Deduced Primary Structure of Cellobiohydrolase II from Trichoderma reesei," Bio/Technology, Vol. 5 (March 1987). Site directed mutagenesis was performed on the CBH II clone and a Bgl II site was placed at the exact 5' end of the open reading frame and a Nhe I site at the exact 3' end. The Bgl II and Nhe I restriction fragment containing CBH II coding sequence was further cloned into a pUC218 phagemid. The CBH II gene was further cut and gel isolated prior to adding a label.

The results of the Northern blot of T. reesei RNA probed with the cbh2 probe indicated that the level of cbh2 specific mRNA reached a peak at 14-18 hours post induction. From this data it can be inferred that the entire cellulase complex including β-glucosidase is induced at this time. The total RNA from 14, 18 and 22 hours is then pooled.

After pooling the specific fractions of total RNA, polyadenylated mRNA is further isolated from the total RNA. Postranscriptional polyadenylation is a common feature of the biogenesis of most eukaryotic mRNAs. The newly synthesized mRNAs have long poly(A) tracts which tend to shorten as mRNAs age. The newly synthesized polyadenylated mRNA is further isolated from total RNA by methods known in the art. These methods include the use of oligo(dT)-cellulose, poly(U) Sepharose, adsorption to and elution from poly(U) filters or nitrocellulose membrane filters, and the like. It is preferable to use oligo(dT) cellulose chromatography in isolating mRNA following the procedure described by Sambrook et al, Molecular Cloning, A Laboratory Manual, 2nd Edition, Cold Spring Harbor Laboratory Press (1989). More specifically, fractions of total RNA are run through the chromatographic resin, and mRNA is eluted therefrom with an elution buffer. The RNA which binds to the column is enriched for RNAs containing poly(A) tails and, therefore, eliminates contaminants, such as rRNA and partially degraded mRNAs. It is important that the purification be carried out successfully such that when cDNA is synthesized from the mRNA, higher yields of mRNA copies and less spurious copying of non-messenger RNAs occurs.

Total RNA and polyadenylated RNA from the preparations were further fractionated on 1% formaldehyde gels, blotted to Nytran^{R} membranes and analyzed to confirm that the enzymes in the cellulase complex were being induced as polyadenylated mRNA.

After isolating polyadenylated mRNA from total RNA, complementary DNA or cDNA is synthesized therefrom. The first strand of cDNA is synthesized using the enzyme RNA-dependent DNA polymerase (reverse transcriptase) to catalyze the reaction. Avian reverse transcriptase which is purified from the particles of an avian retrovirus or murine reverse transcriptase, which is isolated from a strain of E. coli that expresses a cloned copy of the reverse transcriptase gene of the Moloney murine leukemia virus can be used in the present invention. However, it is preferable to use the Moloney murine leukemia virus (M-MLV) reverse transcriptase to synthesize first strand cDNA from the polyadenylated mRNA population. The amount of cloned M-MLV reverse transcriptase required may vary depending on the amount of polyadenylated mRNA used in the synthesis reaction. Usually, about 200 U/µl of the reverse transcriptase is used per 2 to 10 µg of mRNA per reaction.

Also present in the synthesis mixture is a primer to initiate synthesis of DNA. For cloning of cDNAs, any primer can be used, but it is preferable to use oligo(dT) containing 12-18 nucleotides in length, which binds to the poly(A) tract at the 3' terminus of eukaryotic cellular mRNA molecules. The primer is added to the reaction mixture in large molar excess so that each molecule of mRNA binds several molecules of oligo(dT)₁₂₋₁₈. It is preferable to use about 12.5 µg of primer having a concentration of 0.5 mg/ml.

Besides the enzyme and primer, a buffer and dNTP mix containing dATP, dCTP, dGTP, and dTTP at a final concentration of 500 µM each usually completes the reaction cocktail. Any buffer can be used in the present invention for first strand cDNA synthesis that is compatible with this synthesis. It is preferable to use a buffering system consisting of 250 mM Tris-HCl (pH 8.3), 375 mM KCl, 15 mM MgCl₂, and 50 mM dithiothreitol. Generally, about 500 µl of buffer completes the synthesis solution.

After the first strand is synthesized, the second strand of cDNA may be synthesized by a variety of methods known in the art, such as hairpin-primed synthesis by denaturing the cDNA:mRNA complex, adding the Klenow fragment of E.coli DNA polymerase or reverse transcriptase, and then digesting the hairpin loop with nuclease S1 to obtain a double-stranded cDNA molecule, the Okayama and Berg method, the Gubler and Hoffman method, and the like. The Okayama and Berg method uses E. coli RNase H to randomly nick the mRNA, and the RNA is replaced in the nick translation reaction by catalysis with E. coli DNA polymerase I. In the Okayama and Berg method, mRNA is used to prime DNA synthesis by the E. coli DNA polymerase I.

The preferred method to synthesize the second strand of cDNA is a modified method of the Gubler and Hoffman procedure. This procedure uses E. coli RNase H, DNA Polymerase I, and DNA Ligase to form the second strand. Actually, two different methods of proceeding with the second strand synthesis can be used in the present invention. The first procedure uses RNase H to attack the RNA:DNA hybrid in a random fashion, producing nicks in addition to those produced by reverse transcriptase. If too many nicks are introduced into the RNA at the 5' end of the message before second strand synthesis commences, fragments may be produced that are too short to remain hybridized; thus, they will not be able to serve as primers. In addition, the 5'-most RNA oligomer which primes second strand DNA synthesis will continue to be degraded until only two ribonucleotides remain at the 5' end of the second strand DNA. These are substrates for the polymerase I RNase H activity, and the remaining nucleotides will be removed. This leaves the 3' end of the first strand cDNA single stranded, making it a substrate for the 3' exonuclease activity of Polymerase I. The result is a population of cDNAs, which are blunt-ended.

An alternative method relies on M-MLV reverse transcriptase to produce nicks 10 to 20 bases from the 5' end of the RNA in the hybrid. DNA polymerase I is then used for synthesis. Generally, about 500 units at a concentration of 10 U/µl of DNA polymerase I is used. After second strand synthesis, RNase H is added after removal of the DNA polymerase I to produce a duplex, which is entirely DNA, except for the surviving capped RNA 5' oligonucleotide.

The second-strand synthesis by either procedure set forth above usually takes place in the presence of a buffer and dNTP mix. Any buffering system that is known in the art for second strand cDNA synthesis can be used; however, it is preferable to use a buffering system containing 188 mM Tris-HCl, pH 8.3, 906 mM KCl, 100 mM (NH₄)₂SO₄, 46 mM MgCl₂, 37.5 mM dithiothreitol, and 1.5 mM NAD. The dNTP mix preferably contains 10 mM dATP, 10 mM dCTP, 10 mM dGTP, and 10 mM dTTP.

The second strand synthesis is carried out under known procedures set forth in the art The preferred methods and reagents used to synthesize cDNA in the present invention are the BRL cDNA Synthesis System^{R} (Bethesda Research Laboratories, Gaithersburg, Maryland) and the Librarium System (Invitrogen, San Diego, CA).

At this point a pool of cDNAs, a small portion of which code for the bgl1 gene, is present after second strand synthesis. Since amplification of only the specific bgl1 gene fragment in the cDNA pool is crucial for the isolation of the β-glucosidase gene, specific primers were designed to amplify the cDNA fragment encoding the T. reesei bgl1 gene in the polymerase chain reaction (PCR). The primers used are degenerate primers designed to hybridize to the cDNA of the bgl1 gene encoding the N-terminus and an internal CNBr fragment.

In general, it is difficult to isolate the bgl1 gene because the amino acid sequence of the protein does not contain sufficient amino acids which are coded for by unique nucleic acid triplets and thus any oligonucleotide used would be too degenerate to specifically amplify the bgl1 gene in the PCR reaction. However, in this invention, primers were designed by examining the amino acids of the region targeted for amplification of mature β-glucosidase and choosing regions, which will require a reduced degree of degeneracy in the genetic code. Codon bias in T. reesei for various other cellulase genes such as cbh1, cbh2, egl1, and the like was also taken into account when designing the oligonucleotide primers. More specifically, codon bias is based on various genes in the strain T. reesei which display a preferred nucleotide triplet encoding different amino acids. By analyzing this codon bias one can determine that a particular nucleotide sequence coding for an amino acid would be preferred. For example, the cbh1, cbh2 and eg1 genes from T. reesei prefer the CCU coding for the amino acid proline. Thus, when designing an oligonucleotide probe, the CUG sequence would be the preferred choice for leucine, rather than the other triplets (CUU, CUC, CUA, UUA and CUG) which code for leucine.

Furthermore, after selection of an N-terminal region and an internal region as primers for amplification purposes, the primers were designed by inserting a non-specific base inosine into the wobble position of the primer for the N-terminus and using a pool of sixteen variable primer sequences for the internal primer. Basically, the creation of degenerate primers is described by Compton in "Degenerate Primers For DNA Amplification" and Lee et al in "cDNA Cloning Using Degenerate Primers" in PCR Protocols: A Guide to Methods and Applications, published by Academic Press (1990).

Using the primers described above, the cDNA sequences encoding the amino terminal region of the bgl1 gene is then selectively amplified using PCR. The amplification method consists of an initial, denaturing cycle of between about 5 to 15 minutes at 95°C, followed by a 1-7 minutes annealing step at a temperature between 35°C and 55°C and preferably between 45°C and 55°C and a 5-15 minutes polymerization cycle at 65°C. It is preferable, however, to use a 10 minute initial denaturing cycle, followed by 2 minutes of annealing at 50°C and a 10 minute, and preferably a 30 minute polymerization cycle at the aforedescribed temperatures.

The amplified fragment is then identified via gel electrophoresis as a 700 bp cDNA segment. The amplified pool of cDNAs is then further fractionated on a polyacrylamide gel to obtain a more purified 700 bp cDNA fragment for cloning purposes. After elution of the 700 bp fragments from the gel, the 700 bp cDNA fragments are then cloned into phagemid vectors. Any cloning vector can be used to clone the cDNA bgl1 gene fragments, such as pUC18, pUC19, pUC118, pUC119, pBR322, pEMBL, pRSA101, pBluescript, and the like. However, it is preferable to use the cloning vectors pUC218 and pUC219, which are derived from pUC18 and pUC19 by insertion of the intergenic region of M13. The cloning vectors with the cDNA fragments containing the bgl1 gene are then used to transform E. coli strain JM101. After transformation, positive colonies containing the bgl1 gene were identified and DNA isolated therefrom using chloroform:phenol extraction and ethanol precipitation methods.

The nucleotide sequence of the subcloned cDNA 700 bp fragment is then determined by the dideoxy chain termination method described by Sanger et al using a Sequenase^{R} reagent kit provided by U.S. Biochemicals.

From this nucleotide sequence it was determined that the subcloned 700 bp cDNA segment contained an open reading frame encoding 150 amino acids that overlapped a number of other sequenced peptides that were obtained following CNBr and proteolytic degradation of purified T. reesei β-glucosidase. Thus, it was confirmed that the cloned sequences encoded for the extracellular T. reesei β-glucosidase protein.

The cloning of the genomic version of the entire β-glucosidase gene was then undertaken by labelling the 700 bp bgl1 cDNA fragment with ³²P using the methods to label oligonucleotides described by Sambrook et al, supra. This probe is used to identify a 6.0 kb band on a Southern blot of Hind III digested genomic DNA from T. reesei.

The genomic DNA from T. reesei is prepared for Southern blot analysis by deproteinizing the genomic DNA, followed by treatment with ribonuclease A. The prepared genomic DNA is then cut with one of a variety of restriction enzymes such as Eco RI, Hind III and the like, run on a gel, Southern blotted and hybridized with the 700 bp cDNA labelled fragment of the bgl1 gene. From this analysis, it was determined that Hind III was the restriction enzyme of choice that can be used to clone the bgl1 gene.

Hind III is then added to genomic DNA from the strain T. reesei and DNA is extracted therefrom. A sample from this digestion is run on an agarose gel and fractionated electrophoretically. The gel is then Southern blotted and probed with the 700 bp cDNA probe. A 6.0 kb band was then identified on the Southern blot of Hind III digested genomic DNA. The remaining Hind III digested genomic DNA was then subjected to preparative gel electrophoresis and DNA ranging in size from about 5.0 kb to 7.0 kb was eluted therefrom and cloned into a phagemid vector and used to transform E. coli JM101 to create a library. Any phagemid vector can be used such as those described above, however it is preferable to use pUC218. The colonies that resulted from the transformation were then subjected to colony hybridization using the 700 bp cDNA fragment as a probe to identify those colonies that contained the cloned genomic DNA coding for bgl1. The positive colonies from the transformation are then picked and the DNA isolated therefrom by methods known in the art.

The isolated DNA from such a positive colony is then digested with various restriction enzymes, both singly and in various combinations, and subjected to agarose gel electrophoresis. The resultant banding pattern is then used to construct a restriction map of the cloned 6.0 kb genomic DNA from T. reesei. Enzymes used in the digestion include Eco RI, Sst I, Kpn I, Sma I, Bam HI, Xho 1, Bgl II, Cla I, Xba I, Sal I, Pst I, Sph I, Hind III, Bal I, Pvu II and the like.

The same gel is then subject to Southern blot analysis using the same 700 bp bgl1 cDNA as a probe to identify which genomic restriction fragments shared homology with the bgl1 cDNA. Since the position of these homologous fragments can be determined relative to the restriction map of the 6.0 kb genomic fragment and also since the size of the β-glucosidase protein (74 kd) gives an estimated length of the gene as 2.1 kb (because average molecular weight of an amino acid is 105 daltons, a 74 kd protein contains on average 705 amino acids, which in turn is equal to 2,115 bp), then the mapping experiments confirmed that the entire bgl1 gene is contained on the genomic Hind III clone.

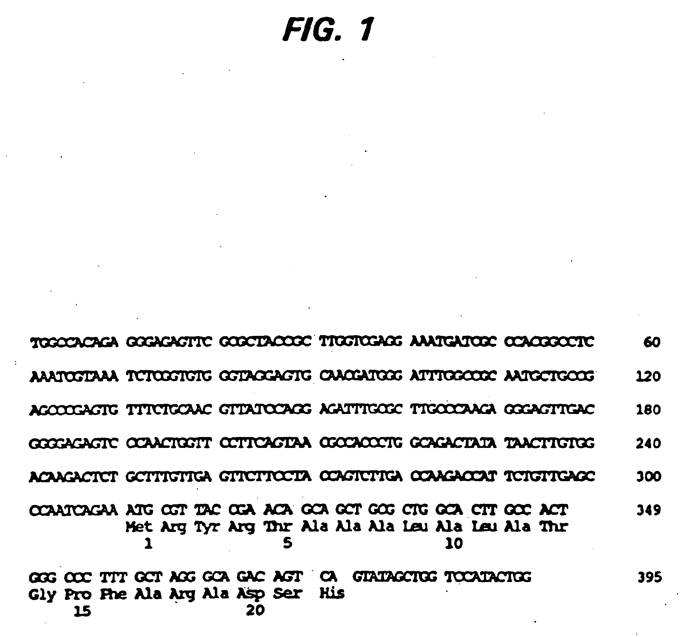

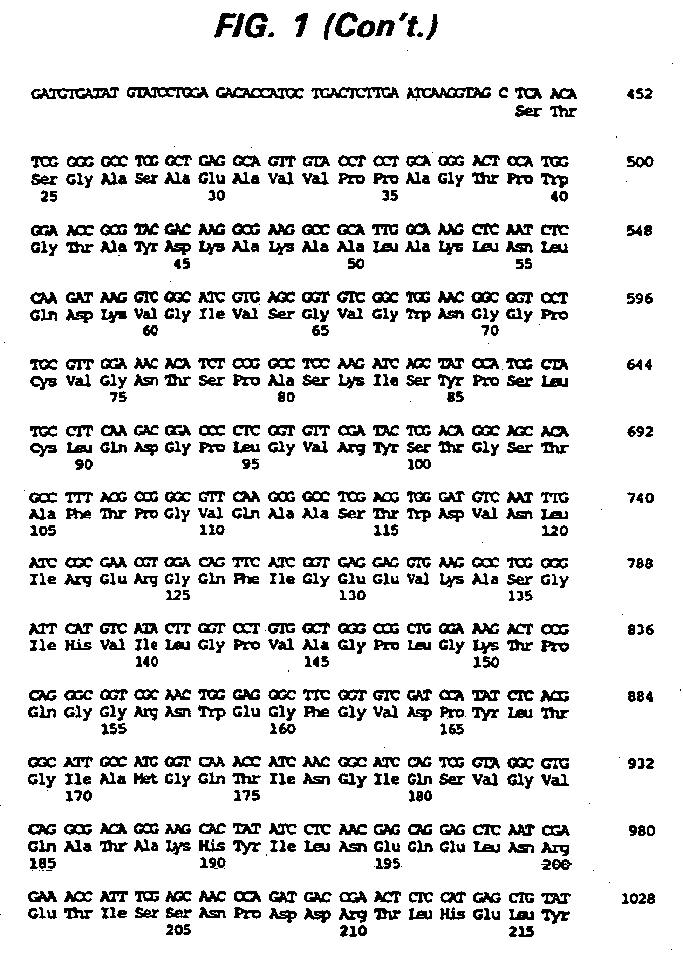

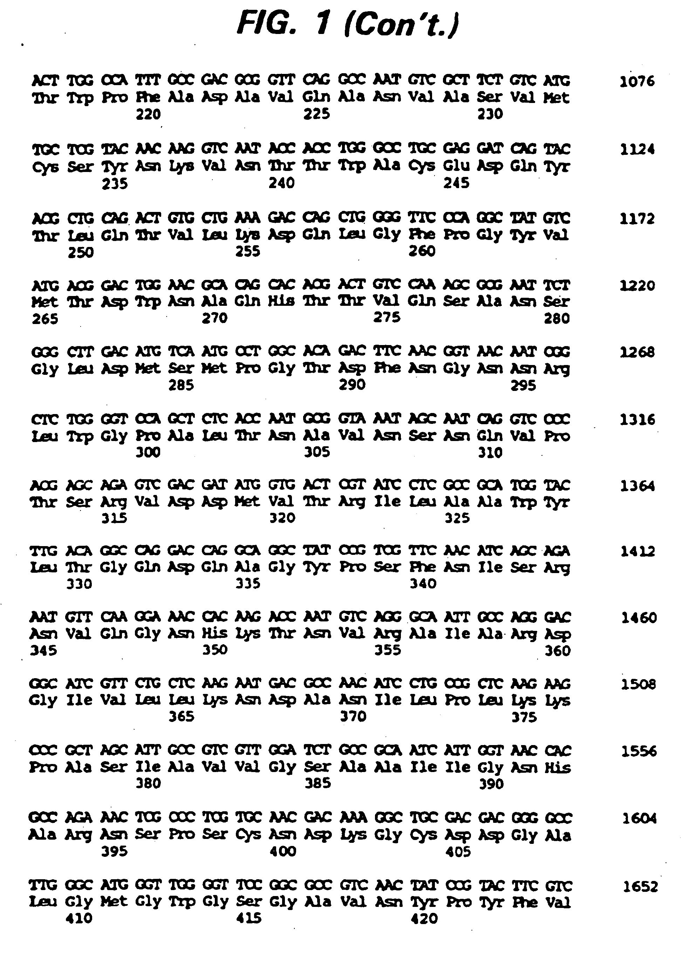

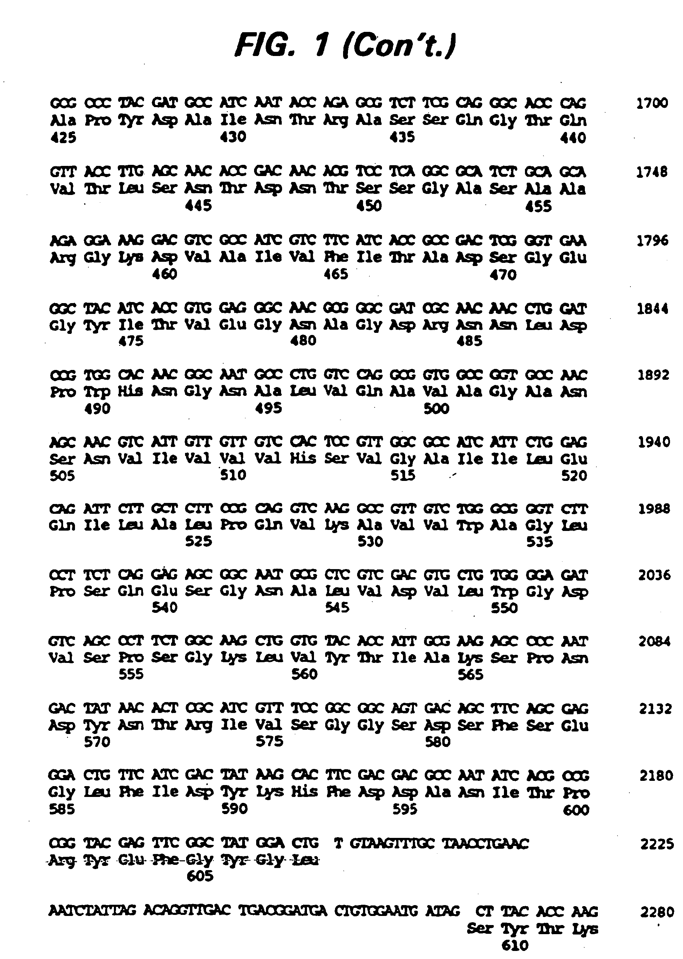

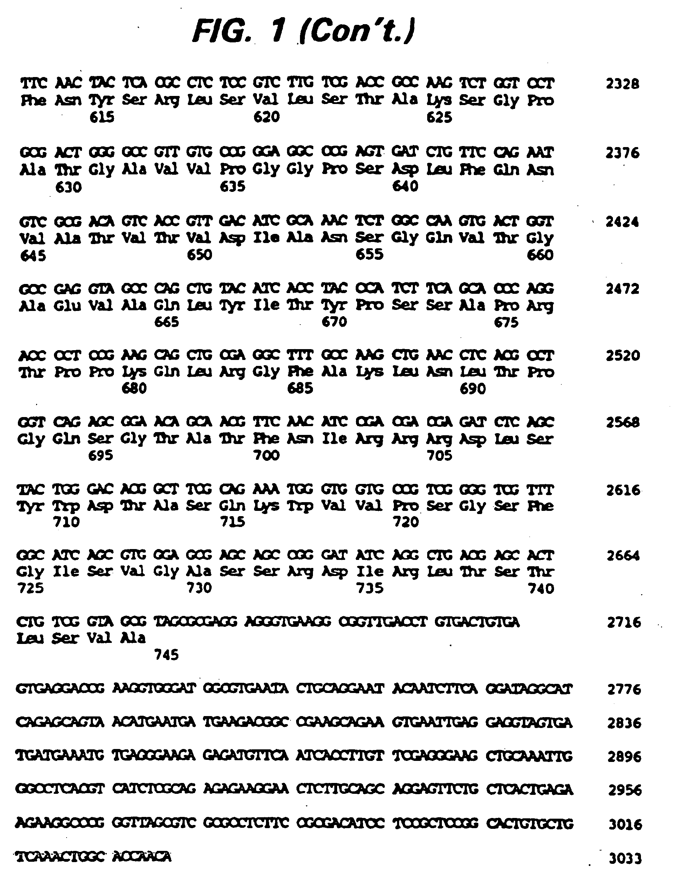

Pvu II and Bal I restriction fragments ranging in size from 600 bp to 1500 bp hybridized with the 700 bp cDNA bgl1 clone and were thus chosen for subcloning into pUC218 phagemids. The nucleotide sequence was determined using the methods of Sanger et al, described above. The Pvu II and Bal I subclones were sequenced and the overlapping sequences of the subclones aligned until a single contiguous sequence totaling 3033 bp was obtained within which the nucleotide sequence of the bgl1 gene was determined on both strands and the position of two small introns was inferred by homology to introns of other genes of filamentous fungi. The amino acid sequence is also deduced as set forth in Figure 1.

The nucleotide sequence and deduced primary amino acid sequence of the entire T. reesei bgl1 gene is set forth in Figure 1. The predicted molecular weight of the encoded β-glucosidase protein is 74,341. A 31 amino acid peptide precedes the mature amino terminus of β-glucosidase as deduced from the amino terminal peptide sequence. Within this peptide, there are three potential signal peptidase recognition sites consisting of Ala-X-Ala.

The primary amino acid sequence of β-glucosidase shows 7 potential N-linked glycosylation sites at positions 208, 310, 417, and 566, which shows the consensus Asn-X-Ser/Thr-X where X is not a proline. However, sites at positions 45, 566, and 658 have a proline residue in the consensus sequence and may or may not be glycosylated.

No unusual codon bias is observed in the bgl1 gene when compared to other cellulase genes. The bgl1 coding region is interrupted by two short introns of 70 bp and 64 bp, respectively. Both introns have splice site donor, splice acceptor, and lariat branch acceptor sites that show homology to the consensus splice signals emerging from T. reesei and other filamentous fungi.

Since the bgl1 gene from the T. reesei strain is identified and can be cloned, the next step is to produce a transformant that has extra copies of the bgl1 gene.

A selectable marker must first be chosen so as to enable detection of the transformed filamentous fungus. Different selectable markers may be used including argB from A. nidulans or T. reesei, amdS from A. nidulans, pyr4 from Neurospora crassa, A. nidulans or T. reesei, and pyrG from Aspergillus niger. The selectable marker can be derived from a gene, which specifies a novel phenotype, such as the ability to utilize a metabolite that is usually not metabolized by the filamentous fungi to be transformed or the ability to resist toxic shock effects of a chemical or an antibiotic. Also contemplated within the present invention are synthetic gene markers that can be synthesized by methods known in the art. Transformants can then be selected on the basis of the selectable marker introduced therein. Because T. reesei does not contain the amdS gene, it is preferable to use the amdS gene in T. reesei as a selectable marker that encodes the enzyme acetamidase, which allows transformant cells to grow on acetamide as a nitrogen source. In the case where the bglI gene is deleted from T. reesei, it is preferable to use the pyrG gene as a selectable marker.

The host strain used should be mutants of the filamentous fungi which lack or have a nonfunctional gene or genes corresponding to the selectable marker chosen. For example, if the selectable marker of argB is used, then a specific arg mutant strain is used as a recipient in the transformation procedure. Other examples of selectable markers that can be used in the present invention include the genes trp, pyr4, pyrG, trp1, oliC31, Bm1, pkiA, niaD, leu, and the like. The corresponding recipient strain must, therefore, be a mutant strain such as trp⁻, pyr⁻, leu⁻, and the like.

The mutant strain is derived from a starting host strain, which is any filamentous fungi strain. However, it is preferable to use a filamentous fungi over-producing mutant strain and particularly, a T. reesei overproducing strain described previously, since this strain secretes high amounts of proteins and, in particular, high amounts of cellulase enzymes. The selected mutant strain is then used in the transformation process. The preferred strain of T. reesei for use in deleting the bglI gene is RLP37 pyrG69, a uridine auxotroph.

The mutant strain of the selected filamentous fungi can be prepared by a number of techniques known in the art, such as the filtration enrichment technique described by Nevalainen in "Genetic improvement of enzyme production in industrially important fungal strains", Technical Research Center of Finland, Publications 26 (1985). Another technique to obtain the mutant strain is to identify the mutants under different growth medium conditions. For instance, the arg⁻ mutants can be identified by using a series of minimal plates supplied by different intermediates in arginine biosynthesis. Another example is the production of pyr⁻ mutant strains by subjecting the strains to fluoroorotic acid (FOA). Strains with an intact pyr4 gene grow in an uridine deficient medium and are sensitive to fluoroorotic acid, and, therefore, it is possible to select pyr4 mutant strains by selecting for FOA resistance.

The chosen selectable marker is then cloned into a suitable plasmid. Any plasmid can be used in the present invention for the cloning of the selectable marker such as pUC18, pBR322, and the like. However, it is preferable to use pUC100. The vector is created by digesting pUC100 with the restriction enzyme SmaI, and the 5' phosphate groups are then removed by digestion with calf alkaline phosphatase. The fragment vector is then purified by gel electrophoresis followed by electroelution from the isolated gel slice. The amdS gene from A. nidulans is isolated as a 2.4 kb SstI restriction fragment following separation of the vector sequences via known procedures such as those described by Hynes et al, Mol. Cell. Biol., 3, pp. 1430-1439 (1983). The 2.4 Kb SstI amdS fragment and the 2.7 Kb pUC100 vector fragment are then ligated together, and the ligation mix is then introduced into the E. coli host strain JM101.

Any plasmid can be used in the present invention for the insertion of the bgl1 gene, but it is preferable to use the pSAS plasmid.

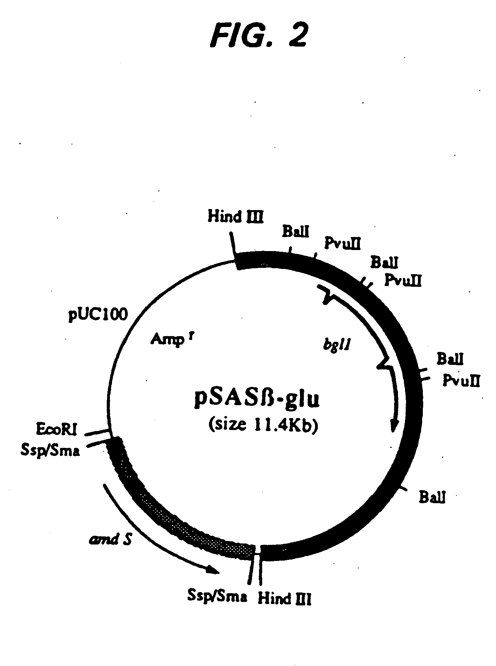

pSASß-glu is constructed by digesting pSAS with the restriction enzyme Hind III and purifying the linear fragment via gel electrophoreses and electroelution. Into this Hind III treated pSAS vector fragment is ligated the 6.0 Kb Hind III fragment of T. reesei genomic DNA that contained all of the coding region of the bgl1 gene along with the sequences necessary for transcription and translation. Figure 2 illustrates the construction of pSASß-glu.

It is also possible to construct vectors that contain at least one additional copy of the bgl1 gene and to construct vectors in which the amino acid sequence of bgl1 gene has been altered by known techniques in the art such as site directed mutagenesis, PCR methods, and chemical mutation methods.

In another embodiment, the bgl1 gene of a β-glucosidic filamentous fungi can be totally deleted and may be replaced with other known genes. Preferably, the replacing gene is homologous to the filamentous fungi so that the resulting recombinant microorganism does not express any heterologous protein. For example, potentially any T. reesei gene which clones for a selected marker and which has been cloned and thus identified in the genome, can replace the bgl1 gene in T. reesei using the techniques described herein.

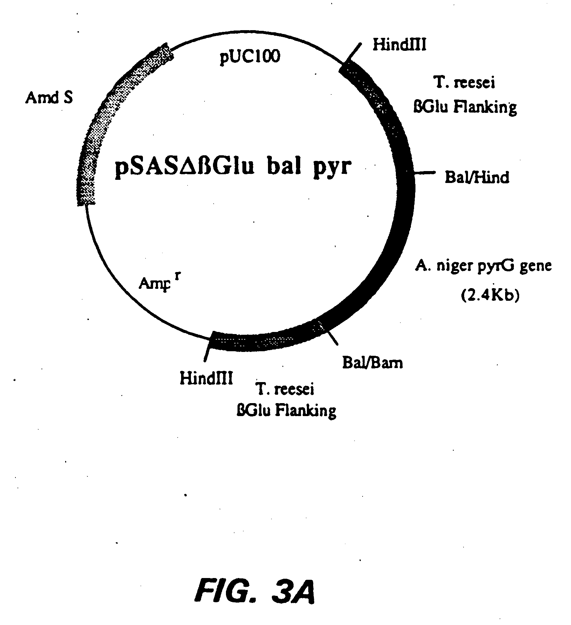

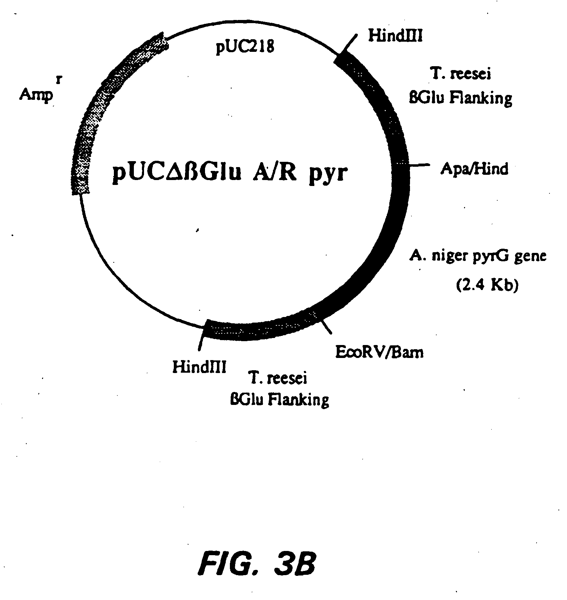

On the other hand, the replacing gene does not necessarily have to be homologous. Specifically, for the deletion of the bglI gene in T. reesei, vectors containing heterologous gene which have been used are illustrated in Figures 3A and 3B. In Figure 3B, a pUC218 vector plasmid having the Aspergillus niger pyrG gene inserted therein is illustrated. A 6.0 Kb genomic Hin dIII fragment, known to contain the entire bgl1 gene, is cloned into the polylinker of pUC218. The coding region for the bgl1 gene is then removed from this plasmid using either unique Bal1 restriction sites (Fig. 3A) or unique ApaI and EcoRV restriction sites (Fig. 3B) situated at the very 5' and 3' end of the bglI open reading frame and replaced with an isolated 2412 bp Hin dIII/Bam HI restriction fragment containing the pyrG gene from Aspergillus niger. All restriction ends are made blunt by treatment with T4 DNA polymerase prior to ligation using T4 DNA ligase.

After a suitable vector is constructed, it is used to transform strains of filamentous fungi. Since the permeability of the cell wall in filamentous fungi (e.g., T. reesei) is very low, uptake of the desired DNA sequence, gene or gene fragment is at best minimal. To overcome this problem, the permeability of the cell wall can be increased or the DNA can be directly shot into the cells via a particle gun approach. In the particle gun approach, the DNA to be incorporated into the cells is coated onto micron size beads and these beads are literally shot into the cells leaving the DNA therein and leaving a hole in the cell membrane. The cell then self-repairs the cell membrane leaving the DNA incorporated therein. Besides this aforedescribed method, there are a number of methods to increase the permeability of filamentous fungi cells walls in the mutant strain (i.e., lacking a functional gene corresponding to the used selectable marker) prior to the transformation process.

One method involves the addition of alkali or alkaline ions at high concentrations to filamentous fungi cells. Any alkali metal or alkaline earth metal ion can be used in the present invention; however, it is preferable to use either CaCl₂ or lithium acetate and more preferable to use lithium acetate. The concentration of the alkali or alkaline ions may vary depending on the ion used, and usually between 0.05 M to 0.4 M concentrations are used. It is preferable to use about a 0.1 M concentration.

Another method that can be used to induce cell wall permeability to enhance DNA uptake in filamentous fungi is to resuspend the cells in a growth medium supplemented with sorbitol and carrier calf thymus DNA. Glass beads are then added to the supplemented medium, and the mixture is vortexed at high speed for about 30 seconds. This treatment disrupts the cell walls, but may kill many of the cells.

Yet another method to prepare filamentous fungi for transformation involves the preparation of protoplasts. Fungal mycelium is a source of protoplasts, so that the mycelium can be isolated from the cells. The protoplast preparations are then protected by the presence of an osmotic stabilizer in the suspending medium. These stabilizers include sorbitol, mannitol, sodium chloride, magnesium sulfate, and the like. Usually, the concentration of these stabilizers varies between 0.8 M to 1.2 M. It is preferable to use about a 1.2 M solution of sorbitol in the suspension medium.

Uptake of the DNA into the host mutant filamentous fungi strain is dependent upon the concentration of calcium ion. Generally, between about 10 mM CaCl₂ and 50 mM CaCl₂ is used in an uptake solution. Besides the need for calcium ions in the uptake solution, other items generally included are a buffering system such as TE buffer (10 mM Tris, pH 7.4; 1 mM EDTA) or 10 mM MOPS, pH 6.0 buffer (morpholinepropane-sulfonic acid), and polyethylene glycol (PEG). The polyethylene glycol acts to fuse the cell membranes, thus permitting the contents of the mycelium to be delivered into the cytoplasm of the filamentous fungi mutant strain and the plasmid DNA is transferred to the nucleus. This fusion frequently leaves multiple copies of the plasmid DNA tandemly integrated into the host chromosome. Generally, a high concentration of PEG is used in the uptake solution. Up to 10 volumes of 25% PEG 4000 can be used in the uptake solution. However, it is preferable to add about 4 volumes in the uptake solution. Additives such as dimethyl sulfoxide, heparin spermidine, potassium chloride, and the like may also be added to the uptake solution and aid in transformation.

Usually a suspension containing the filamentous fungi mutant cells that have been subjected to a permeability treatment or protoplasts at a density of 10⁸ to 10⁹/ml, preferably 2 x 10⁸/ml, are used in transformation. These protoplasts or cells are added to the uptake solution, along with the desired transformant vector containing a selectable marker and other genes of interest to form a transformation mixture.

The mixture is then incubated at 4°C for a period between 10 to 30 minutes. Additional PEG is then added to the uptake solution to further enhance the uptake of the desired gene or DNA sequence. The PEG may be added in volumes of up to 10 times the volume of the transformation mixture, preferably, about 9 times. After the PEG is added, the transformation mixture is then incubated at room temperature before the addition of a sorbitol and CaCl₂ solution. The protoplast suspension is then further added to molten aliquots of a growth medium. This growth medium contains no uridine and selectively permits the growth of transformants only. The subsequent colonies were transferred and purified on a growth medium depleted of sorbitol.

At this stage, stable transformants can be distinguished from unstable transformants by their faster growth rate and the formation of circular colonies with a smooth rather than ragged outline on solid culture medium. Additionally, in some cases, a further test of stability can be made by growing the transformants on solid non-selective medium, harvesting the spores from this culture medium and determining the percentage of these spores which will subsequently germinate and grow on selective medium.

In order to ensure that the transformation took place by the above-described methods, further analysis is performed on the transformants such as Southern blotting and autoradiography. Using the same basic procedures set forth above, the entire bgl1 gene can be deleted from a vector and transformed into filamentous fungi strains or the bgl1 gene can be altered and transformed into filamentous fungi strains.

After confirmation that the transformed strains contained at least one additional copy of the bgl1 gene, an altered bgl1 gene or the transformants contained a deleted bgl1 gene, the strains are further cultured under conditions permitting these transformants to propagate. The transformants can then be isolated from the culture media and used in a variety of applications which are described below. Alternatively, the transformants can be further fermented and a recombinant fungal cellulase composition can be isolated from the culture media. Since, for example, the transformants described herein can express enhanced, deleted or altered extracellular β-glucosidase in the fermentation medium, fungal cellulase compositions can be isolated from the medium. Usually, the isolation procedure involves centrifuging the culture or fermentation medium containing the transformants and filtering by ultrafiltration the supernatant to obtain a recombinantly produced fungal cellulase composition. Optionally, an antimicrobial agent can be further added to the composition prior to use in the variety of applications described below. Examples of microbial agents that can be added are sodium azide, sodium benzoate and the like.

Confirmation that the transformants produced by the process had enhanced activity on cellobiose, the following experiment was performed. In this experiment 50 mg of cellobiose which was suspended in 1.0 ml of phosphate buffer (pH 5.0) and was reacted with the fermentation product produced by the transformant (65.5 mg/ml protein) using a fermentation product from a normal nonmutant T. reesei strain as a control (135.0 mg/ml protein). The results of cellobiase activity under conditions of initial rate, are set forth in Table I below:

**TABLE I**

| Product | Protein (mg/ml) | Activity on Cellobiose µmole glucose mg protein |
|---|---|---|
| Control | 135.0 | 6 |
| Product produced by the present invention | 65.5 | 33 |

The results from this experiment indicate that the fermentation product produced by the transformants has over five times the specific activity on the substrate, cellobiose, compared to a nonmutant T. reesei control strain.

In one embodiment of this invention, the deletion of the bgl1 gene from T. reesei strains would be particularly useful in preparing cellulase compositions for use in detergents and in isolating cellooligosaccharides (e.g., cellobiose).

The cellulase enzymes have been used in a variety of detergent compositions to enzymatically soften clothes and to provide color restoration. However, it is known in this art that use of cellulase enzymes can impart degradation of the cellulose fibers in clothes. One possibility to decrease the degradation effect is to produce a detergent that does not contain β-glucosidase. Thus, the deletion of this protein would effect the cellulase system to inhibit the other components via accumulation of cellobiose. The modified microorganisms of this invention are particularly suitable for preparing such compositions because the bgl1 gene can be deleted leaving the remaining CBH and EG components thereby resulting in color restoration and improved softening benefits in the composition without degradative effects.

The detergent compositions may employ, besides the cellulase composition (deleted in β-glucosidase), a surfactant, including anionic, nonionic and ampholytic surfactants, a hydrolase, building agents, bleaching agents, bluing agents and fluorescent dyes, caking inhibitors, solubilizers, cationic surfactants and the like. All of these components are known in the detergent art. For a more thorough discussion, see U.S. Application Serial No. 07/593,919 entitled "Trichoderma reesei Containing Deleted Cellulase Genes and Detergent Compositions Containing Cellulases Derived Therefrom", and U.S. Serial No. 07/770,049, filed October 4, 1991 and entitled. "Trichoderma reesei Containing Deleted and/or enriched Cellulase and other enzyme Genes and Cellulase Compositions Derived Therefrom" both of which are incorporated herein by reference in their entirety.

Preferably the cellulase compositions are employed from about 0.00005 weight percent to about 5 weight percent relative to the total detergent composition. More preferably, the cellulase compositions are employed from about 0.01 weight percent to about 5 weight percent relative to the total detergent composition and even more preferably, from about 0.05 to about 2 weight percent relative to the total detergent composition.

Deletion of the bgl1 gene would also provide accumulation of cellooligosaccharides (e.g., cellobiose) in cellolosic solutions treated with cellulase system, which can be purified therefrom. In this regard, the present invention presents the possibility to isolate cellooligosaccharides employing microorganisms in an easy and effective manner.

Cellooligosaccharides are useful in assaying cellulase enzymes for enzymatic activity and are also useful in the synthesis of ethanol and glucose. Moreover, it is contemplated that such oligosaccharides would also be useful as food additives, chemical intermediates, etc.

Heretofore, the use of cellulase containing β-glucosidase to prepare cellooligosaccharides required the deactivation of β-glucosidase by adjusting the pH of the solution to less than about 4 and generally to around 3.8. At this pH, the β-glucosidase is generally inactivated. However, at this pH, the other enzyme components of cellulase are generally less active as compared to their optimum pHs and, accordingly, such a reduction of pH to inactivate the β-glucosidase necessarily results in a less efficient process.

On the other hand, the use of cellulase compositions free of β-glucosidase as per this invention provides a facile means for preparing cellooligosaccharides at a pH of from about 4.5 to about 8; preferably at a pH of from about 4.5 to about 6 and most preferably at the pH optimum for the cellulase composition employed. In this embodiment, provided herein is a process for producing cellooligosaccharides which comprises contacting cellulose containing materials (i.e., materials containing at least 20% cellulose and preferably at least 50% cellulose) with a cellulase composition free of β-glucosidase at a pH of from about 4.5 to about 8. Additionally, cellulase compositions containing reduced amounts of β-glucosidase can be obtained by mixing the cellulase produced by a β-glucosidic filamentous fungi and the cellulase produced by a β-glucosidic filamentous fungi which has been modified to be incapable of producing β-glucosidase.

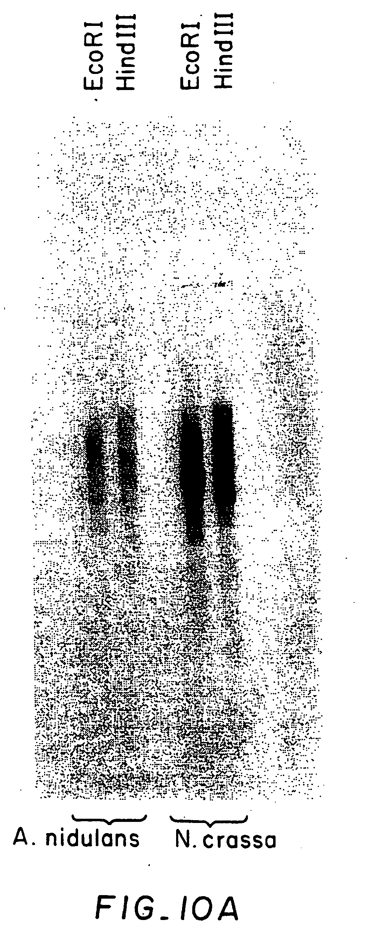

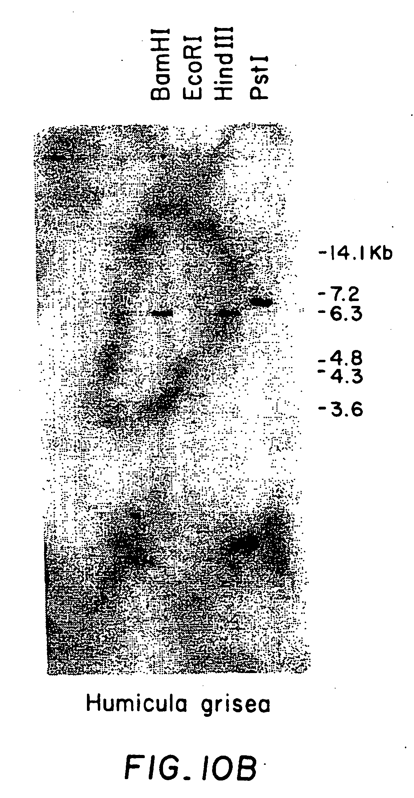

Also described herein is the use of the β-glucosidase nucleotide sequence of T. reesei to design various probes for the identification of the extracellular β-glucosidase gene in other filamentous fungi. In this regard, the entire nucleotide sequence of the bgl1 gene can be used or a portion thereof to identify and clone out the equivalent genes from other filamentous fungi. The sources of filamentous fungi include those fungi from the genus Trichoderma, Aspergillus, Neurospora, Humicola, Penicillium and the like. More particularly, the preferred species include Trichoderma reesei, Trichoderma viridae, Aspergillus niger, Aspergillus oryzae, Neurospora crassa, Humicola grisea, Humicola insolens, Penicillium pinophilum, Penicillium oxalicum, Aspergillus phoenicis, Trichoderma koningii and the like. Due to the species homology of the bgl1 gene, filamentous fungi equivalent genes are easily identified and cloned. Indicative of this are Figures 10A and 10B which illustrate autoradiograph of A. nidulans and N. crassa (Figure 10A) and H. grisea (Figure 10B) DNA digested with Hind III and Eco RI and further were blotted and probed with a P³² labeled Hind III 6.0 kb bgl1 DNA fragment containing the bgl1 gene of T. reesei. These autoradiographs clearly illustrate that a DNA fragment containing the bgl1 gene of T. reesei can be used to identify the extracellular bgl1 gene in other fungi.

Thus the bgl1 gene of other filamentous fungi may be cloned by the methods outlined above using the P³² labelled T. reesei bgl1 gene as a probe. Once the genes of other filamentous fungi are cloned, they can be used to transform the filamentous fungi from which the gene was derived or other filamentous fungi to overproduce β-glucosidase by the methods described above. Alternatively, the cloned bgl1 genes from the other filamentous fungi can be used by the methods described above to delete or disrupt the bgl1 gene in the genome of the filamentous fungi from which the bgl1 gene was originally cloned.

In order to further illustrate the present invention and advantages thereof, the following specific examples are given.

### Example 1

### Isolation of Total RNA from Trichoderma reesei

A Trichoderma reesei culture which over produces cellulases was specifically induced for cellulase using sophorose, a β,1-2 diglucoside as described by Gritzali, 1977. The starting strain of Trichoderma reesei is a cellulase over-production strain (RL-P37) developed by mutagenesis by the methods described by Sheir-Neiss, G. and Montenecourt, B.S., Appl. Microbiol. Biotechnol., Vol. 20 (1984) pp. 46-53. A mycelial inoculum of T. reesei, from growth on potato dextrose agar (Difco), was added into 50 ml of Trichoderma basal medium containing 1.40 grams/liter (NH₄)₂·SO₄, 2.0 grams/liter KH₂PO₄, 0.30 grams/liter MgSO₄, 0.30 grams/liter urea, 7.50 grams/liter BactoPeptone, 5.0 ml/liter, 10% Tween - 80, 1.0 ml/liter trace elements-EFG, pH 5.4, which was filtered through a 0.2 micron filter in a 250 ml baffled flask. This culture was incubated at 30°C for 48 hours with vigorous aeration. Five milliliter aliquots were taken from the culture and added to 25 ml of fresh basal medium in seven 250 ml flasks. These were subsequently grown for 24 hours at 30°C. All cultures were centrifuged in a benchtop clinical centrifuge at 2400 x g for 10 minutes. The mycelial pellets were washed three times in 50 mls of 17 mM KHPO₄ buffer (pH 6.0). Lastly, the mycelia were suspended in six flasks containing 50 ml of 17 mM KHPO₄ buffer with the addition of 1 mM sophorose and a control flask containing no sophorose. The flasks were incubated for 18 hours at 30°C prior to harvesting by filtration through Miracloth (Calbiochem). The excess medium was then squeezed out and the mycelial mat was placed directly into liquid nitrogen and may be stored at -70°C for up to one month. The frozen hyphae were then ground in an electric coffee grinder that was prechilled with a few chips of dry ice until a fine powder was obtained. The powder was then added to about 20 ml of an extraction buffer containing 9.6 grams of p-aminosalicylic acid dissolved in 80 ml of DEP-treated water, 1.6 grams of triisopropylnaphthalene sulfonic acid dissolved in 80 ml of DEP-treated water, 24.2 grams Tris-HCl, 14.6 grams NaCl, 19.09 grams EDTA, which was diluted to 200 ml total volume with DEP-treated water and the pH was adjusted to 8.5 with NaOH. After addition of the extraction buffer, 0.5 volumes of TE-saturated phenol was also added thereto, and the extraction mixture was placed on ice. One quarter volume of chloroform was then added to the extraction mixture, and the mixture was shaken for two minutes. The phases were then separated by centrifugation at 2500 rpm. The aqueous phase was removed and placed in a centrifuge tube, which contained a few drops of phenol in the bottom of said tube. The tube was placed on ice. The organic phase was then reextracted with 2.0 ml of extraction buffer and placed in a 68°C water bath for 5 minutes to release the RNA trapped in polysomes and at the interface of the extraction mixture. The extracted mixture was then centrifuged, and the aqueous phase removed and pooled with the other aqueous fraction.

The entire aqueous fractions were then extracted with phenol-chloroform (1:1 v/v) for 4 to 5 times until there was no longer any protein seen visually at the interface. Then 0.1 volume of 3 M sodium acetate, pH 5.2 (made with DEP water and autoclaved) and 2.5 volumes of 95% was added to the organic extracts, and the extracts were frozen at -20°C for 2 to 3 hours. Alternatively, the RNA was precipitated using 2 M lithium acetate. The RNA was then pelleted by centrifugation at 12,000 rpm for 20 minutes. The pelleted RNA was then resuspended in DEP-water with an RNase inhibitor to a final concentration of 1 unit per µl. To determine whether the genes encoding the enzymes were being induced, total RNA was analyzed.

### Analysis of Total RNA Preparation

To confirm whether the genes encoding the enzymes of the cellulase complex were being induced, total RNA was analyzed by Northern blotting as described by Sambrook et al, supra using a P³² fragment of the T. reesei cbh2 gene as a probe. The cbh2 clone was isolated using the methods described by Chen et al in "Nucleotide Sequence and Deduced Primary Structure of Cellobio-hydrolase II from Trichoderma reesei", Biotechnology, Vol. 5 (March 1987), incorporated herein by reference. Site directed mutagenesis (Sambrook et al., supra) was performed on the cbh2 clone and a Bgl II site was placed at the exact 5' end of the opening reading frame and an Nhe I site at the exact 3' end. The Bgl II/Nhe I coding sequence was then cloned into a pUC218 phagemid. For use as a probe, the cbh2 fragment was digested with Bgl II/Nhe I and isolated by gel electrophoresis. The results indicated that the level of cbh2 specific mRNA reached a peak at 14-18 hours post induction. The total RNA from 14, 18 and 22 hours was then pooled.

### Example 2

### Purification of Polyadenylated mRNA

mRNA was then isolated from the pooled fraction of total RNA set forth above using oligo (dT) cellulose chromatography. Oligo(dT) cellulose (type 3 from Collaborative Research, Lexington, MA) is first equilibrated with Oligo(dT) binding buffer containing 0.01 M Tris-HCl, pH 7.5, 0.5 M NaCl, and 1 mM EDTA, then aliquots of 25-300 mg were added to 1.5 ml microfuge tubes. RNA dissolved in 1 ml of binding buffer was added and allowed to bind for 15 min. with gentle shaking. The suspensions were centrifuged at 1500 g for 3-5 min., washed 3-5 times with 1 ml of binding buffer, and then washed 3 times with 400 µl of elution buffer containing 0.01 M Tris-HCl, pH 7.5, and 1 mM EDTA. The eluates were pooled, readjusted to 0.5 M NaCl, rebound, and reeluted with three washes of elution buffer. The final three elution buffer washes were pooled and mRNA was recovered by ethanol precipitation.

### Analysis of Total RNA and polyadenylated mRNA

Total RNA and the polyadenylated RNA were fractionated on 1% formaldehyde-agarose gels using 10 µg of RNA for each lane, blotted to Nytran^{R} membranes and analyzed by the Northern blot method described by Thomas in "Hybridization of denatured RNA and Small DNA fragments transferred to Nitrocellulose", Proc. Natl. Acad. Sci. USA, Vol. 77 (1980), pp. 5201-5205.

Briefly, this procedure involves denaturing RNA (up to 10 µg/8 µl reaction) by incubation in 1 M glyoxal/50% (vol/vol) Me₂SO/10 mM sodium phosphate buffer pH 7.0 at 50°C for 1 hr. The reaction mixture was cooled on ice and 2 µl of sample buffer containing 50% (vol/vol) glycerol, 10 mM sodium phosphate buffer at 7.0 and bromophenol blue was added. The samples were electro-phoresed on horizontal 1% formaldehyde-agarose gels in 10 mM phosphate buffer, pH 7.0 at 90 v for 6 hours.

The glyoxylated RNA was transferred from agarose gels to nitrocellulose by using 3 M NaCl/0.3 M trisodium citrate (20X NaCl/cit). After electrophoresis, the gel was placed over two sheets of Whatman 3 MM paper which was saturated with 20X NaCl/cit. Nitran^{R} membrane was wetted with water, equilibrated with 20X NaCl/cit and laid over the gel. The gel was then covered with two sheets of Whatman 3 MM paper and a 5 to 7 cm layer of paper towels, a glass plate and a weight. Transfer of the RNA was completed in 12-15 hours. The blots were then dried under a lamp and baked in a vacuum for over 2 hrs. at 80°C.

The membranes were probed with a cbh2 probe to verify that the polyadenylated mRNA pool contained cbh2 mRNA and by inference the genes encoding the enzymes of the cellulase complex were indeed induced.

### Example 3

### Synthesis of cDNA

### A. First Strand Synthesis

Synthesis of cDNA was performed using the BRL cDNA Synthesis System^{R} (Bethesda Research Laboratories, Md.) according to the instructions of the manufacturer. To a sterile, DEPC-treated tube in ice was added 10 µl of 5X First Strand Buffer containing 250 mM Tris-HCl, pH 8.3, 375 mM KCl, 15 mM MgCl₂, 50 mM DTT, 2.5 µl 10 mM dNTP Mix (10 mM dATP, 10 mM dCTP, 10 mM dGTP, 10 mM dTTP), 5 µl Gligo (dT)₁₂₋₁₈ (0.5) mg/ml), 10 µl of mRNA at 0.5 mg/ml and 20 µl diethylpirocarbmate(DEPC)-treated water to create a final composition containing 50 mM Tris-HCl (pH 8.3), 75 mM KCl, 3 mM MgCl₂, 10 mM dithiothreitol, 500 µM each dATP, dCTP, dGTP and dTTP, 50 µg/ml Oligo (dT)₁₂₋₁₈, 100 µg/ml polyadenylated RNA and 10,000 U/ml cloned M-MLV reverse transcriptase. A control run was also run simultaneously using 10 µl of a 2.3 kb control RNA (0.5 mg/ml) in lieu of the mRNA.

The reaction was initiated by adding 2.5 µl of Molony murine leukemia virus (M-MLV) reverse transcriptase (100 Units/µl) to the mRNA tube and the control RNA. The samples were mixed. All reaction tubes were incubated at 37°C for one hour and then placed on ice.

A small aliquot from the reaction mixture was run on a gel to confirm its presence and quantity. The yield obtained was about 2-6 µg.

### B. Second Strand Synthesis

To the control tube on ice after first strand synthesis was added 230.6 µl DEPC-treated water, 6 µl 10 mM dNTP mix, 32 µl 10X second strand buffer containing 188 mM Tris-HCl, pH 8.3, 906 mM KCl, 100 mM (NH₄)₂ SO₄, 46 mM MgCl₂, 37.5 mM dithiothreitol, 1.5 mM NAD, 8 µl E. coli DNA Polymerase I (10 µ/µl), 1.4 µl E. coli RNase H and 1 µl E. coli DNA ligase (100 units).

To the first strand synthesis of the sample was added on ice 289.5 µl of DEPC-treated water, 7.5 µl 10 mM dNTP mix, 40 µl 10X second strand buffer, 10 µl E. coli DNA Polymerase I, 1.75 µl E. coli RNaseH and 1.25 E. coli DNA ligase, to create a final composition containing 25 mM Tris-HCl (pH 8.3), 100 mM KCl, 10 mM (NH₄)₂SO₄, 5 mM MgCl₂, 250 µM each dATP, dCTP, dGTP, dTTP, 0.15 mM NAD, 5 mM Dithiothreitol, 250 U/ml DNA Polymerase I, 8.5 U/ml RNase H, and 30 U/ml DNA Ligase. Both the control tube and the sample tube were vortexed gently and incubated for 2 hours at 16°C. After incubation, both tubes were placed on ice.

The sample tube was then extracted with 415 µl of phenol and ethanol precipitated. The pellet was dissolved in 200 µl of sterile TE buffer (10 mM Tris-HCl pH 7.5, 1 mM Na₂EDTA) and reprecipitated from 7.5 M ammonium acetate with ethanol.

An aliquot of the sample was further analyzed by gel electrophoresis to check for purity. The yield of the synthesis was about 4.0 µg.

The remaining control sample was further extracted with phenol and ethanol precipitated as described above for the sample. After dissolving the pellet in 200 µl of sterile TE buffer, reprecipitating the sample from ammonium acetate with ethanol, and redissolving the dry pellet in 20 µl of sterile TE buffer, 2 µl of the solution was then further analyzed by gel electrophoresis to check for purity.

### Example 4

### Amplification of bgl1 cDNA Sequences

Amplification of the cDNA fragments encoding a portion of the T. reesei β-glucosidase gene, bgl1, was performed using the polymerase chain reaction (PCR) method with Taq^{R} polymerase and a Perkin Elmer Cetus Thermal Cycler^{R}.

The reaction mixture was formed by mixing 76 µl deionized water, 10 µl of a 10X mixture of buffer containing 166 mM (NH₄)₂ SO₄, 670 mM Tris-HCl, pH 8.8, 67 mM MgCl₂, 67 µm EDTA, 10 mM β-mercaptoethanol, 10 µl dimethylsulfoxide and 1.7 mg/ml BSA diluted to a total volume of 1.0 ml with deionized water, 8 µl of 2 dNTPs (each), 1 µl 5' oligonucleotide primer, 1 µl 3' internal oligonucleotide primer, 1.0 µg cDNA diluted in 3 µl deionized water, and 1 µg Taq^{R} polymerase.

The amplification method consists of an initial denaturing cycle at 95°C for 10 minutes, followed by a two minute annealing step at 50°C and a 10 minute polymerization cycle at 65°C, for an additional 30 cycles.

### A. Oligonucleotide Primers

The oligonucleotide primers used to amplify the cDNA fragment encoding the T. reesei bgl1 gene were designed based on the degeneracy of the genetic code for the selected amino acids for an N terminal region of the bgl1 gene and an internal oligonucleotide. The 5' oligonucleotide primer consisted of the sequence: 5' GCI GTI CCT CCT GCI GG 3' wherein I = inosine.

The internal 3' oligonucleotide primer consisted of a pool of 16 x 21 oligonucleotides. This pool was based on various derivations of the following sequences:

5' GTT G/ATT ICC G/ATT G/AAA G/ATC TGT 3'

### Example 5

### Subcloning of PCR Generated Fragments

Ninety µl of each reaction mix was fractionated on 4% polyacrylamide gels in 1X TBE, the major band was excised and eluted from the gel slice as described by Sambrook et al, supra. The eluted DNA fragment was precipitated in ethanol and resuspended in 15 µl of TE buffer (10 mM Tris, 1 mM EDTA). Each 1-2 µg DNA fragment was then treated with 0.5 mM ATP and T₄ polynucleotide kinase to phosphorylate the 5' end of each fragment following by the procedures of Sambrook et al, supra. Blunt ends were generated by adding 3 µl of 10X T₄ polymerase buffer (330 mM Tris-acetate at pH 7.9, 660 mM potassium acetate, 100 mM magnesium acetate, 1 µl of 2.5 mM dNTPS, 1 µl of T₄ DNA polymerase and 5 µl of distilled water). The blunt-end reaction mixture was then incubated at 37° for 60 minutes. The reaction was-stopped by addition of EDTA to a final concentration of 1 mM EDTA and the sample was further heated for 10 minutes at 65°C.

The blunt-end DNA fragments were then ligated with SmaI cleaved and dephosphorylated pUC218 which had been infected with M13X07 as described by Sambrook et al, supra. The cloning vectors pUC218 and pUC219 were derived from pUC118 and pUC119 by insertion of the Bgl II, Cla I and Xho I polylinker as described by Korman et al in "Cloning, Characterization, and expression of two α-amylase genes from Aspergillus niger var. awamori", Current Genetics, Vol. 17, pp. 203-212, (1990).

The aforedescribed phagemid was then used to transform E. coli strain JM101 as described by Yarnisch et al in "Improved M13 phage cloning vectors and host strains: nucleotide sequence of the M13mp18 and pUC19 Vectors", Gene, Vol. 1197, pp. 103-119 (1985).

### Example 6

### Isolation of cDNA Subcloned Fragment

The transformed strain was inoculated in 1.5 ml of 2YT broth in a tube which had been previously inoculated with 15 µl of saturated E. coli JM101. The culture was grown for 8 hours under shaking at 37°C.

The culture mixture was then spun at 6000 rpm for 5 minutes, and the supernatant was poured off into another tube. To the supernatant 300 µl of 2.5 M NaCl, 20% PEG was added, and the solution was mixed. The mixture was then incubated at room temperature for 15 minutes.

The solution was then spun for 5 minutes in a microfuge, and the supernatant was aspirated off. The solution was vortexed once again, and the supernatant was further aspirated off.

100 µl of equilibrated phenol was added to the tube, and the tube was vortexed. 100 µl of chloroform was added, and once again the tube was vortexed. The tube was heated at 55°C for 5 minutes, mixed, and microfuged an additional 5 minutes.

160 µl of the supernatant was then pipetted off and transferred to a clear tube. 20 µl of 1N NaOAC, pH 4.5, and 400 µl of 95% ETOH were added to the supernatant, and the solution was mixed and frozen on dry ice for 5 minutes. The tube was then spun for an additional 15 minutes, and the supernatant was aspirated off.

1000 µl of 70% ethanol was added to the tube, and the tube was spun for an additional 2 minutes and reaspirated. The mixture was spun once more under vacuum for 4 minutes, and the pellet was resuspended in 15 µl TE buffer.

### Example 7

### Determination of the Nucleotide Sequence of 700 bp cDNA fragment

The nucleotide sequence of the 700 bp cDNA fragment was determined using the dideoxy DNA sequencing method described by Sanger et al, "DNA Sequencing with chain terminating inhibitors", Proc. Natl. Acad. Sci. U.S.A., Vol. 74 (1977), p. 5463, using the Sequenase^{R} reagent kit (U.S. Biochemicals).

### Example 8

### Analysis of bgl1 gene

### A. Sequence Analysis

Nucleotide sequencing was done by the dideoxy chain termination method of Sanger et al (1977) using the Sequenase^{R} reagent kit (U.S. Biochemicals).

### B. Amino Acid Sequencing

A 2.5-nmol sample of the reduced and carboxymethylated β-glucosidase preparation purified (per Chirico and Brown, European Journal of Biochem., Vol. 165, pp. 333 et seq.) was subjected to N-terminal sequencing on a proprietary multiphase sequencer.

To a sample of β-glucosidase, Endo-Lys C protease was added to 1% of the total protein and the mixture incubated for 1 hour at 37°C or the protein sample was subject to cyanogen bromide treatment. An equal volume of HPLC solution A (0.05% TEA/0.05% TFA in water) was added to stop the reaction. The resulting CNBr and Endo-Lys C fragments were separated by chromatography on a Brownlee C-4 column using a linear gradient of 0-100% HPLC solution B (0.05% TEA/0.05% TFA in n-propanol) at a rate of 1% per minute. Several peaks were collected for amino acid sequencing and the data are denoted in Fig. 1.

### Example 9

### Identification of bgl1 gene from T. reesei

The 700 bp bgl1 cDNA fragment was then labelled with ³²P using methods described by Sambrook et al, supra.

Genomic DNA from T. reesei was prepared by filtering a 24-36 hour culture of T. reesei through Miracloth and freezing the mycelia obtained from the culture medium. The frozen mycelia were then ground into a fine powder and 22 mls of TE, and 4 mls of 20% SDS were added to the powdered mycelia and mixed. 10 ml of phenol and chloroform was added to the mixture prior to centrifugation and removal of the aqueous phase. 200 µl of 5 mg/ml proteinase K was added to the organic extract, and the mixture was incubated for 20 minutes at 55°C. The DNA was then further extracted by methods known in the art using chloroform/phenol extraction followed by ethanol precipitation. The isolated DNA was then treated with 1 µg of heated ribonuclease A (100°C for 15 minutes) per 20 µg of genomic DNA in TE buffer at 37°C for 30 minutes, then cooled to room temperature. The genomic DNA from T. reesei was then cut singly or in combination with a variety of restriction enzymes such as Eco RI, Hind III and the like, Southern blotted and hybridized with the P³² labelled 700 bp cDNA fragment of the bgl1 gene as a probe. From this analysis it was determined that Hind III was the restriction enzyme of choice used to locate the β-glucosidase gene.

10 to 20 units of Hind III per milligram of genomic DNA was added to the DNA and then the DNA was extracted with phenol-chloroform to remove protein. The treated DNA was then alcohol precipitation and resuspended to 2 grams/liter in TE buffer.

4 µl samples from the Hind III digestion of genomic DNA were loaded on a 1% agarose gel and fractionated electrophoretically. The gel was then Southern blotted and probed with the P³² 700 bp cDNA probe. A 6.0 kb band was identified on the Southern blot of Hind III digested genomic DNA from T. reesei.

The remaining Hind III genomic DNA was then subjected to a preparative gel electrophoresis and fragments ranging from 5 kb to 7 kb were then electroeluted from the agarose gel and cloned into Hind III digested pUC218. The resulting plasmids were used to transform E. coli JM101 to create a library. Then the library was screened by colony hybridization using P³² labelled 700 bp bgl1 cDNA as a probe to identify those colonies containing DNA coding for the bgl1 gene.

The positive colonies from the transformation were then picked and the DNA isolated therefrom by phenol:chloroform extraction and ethanol precipitation, described by Sambrook et al, supra.

The isolated DNA from the positive colonies was digested both singly and in various combinations with the following restriction enzymes: Hind III, Eco RI, Sst 1, Kpn I, Bam HI, Xho 1, Bgl II, Cla I, Xba I, Sal I, Pst I, Sph I, Bal I, and Pvu II. The digestions were subjected to agarose gel electrophoresis, and the resultant banding pattern was used to construct a restriction map of the cloned 6.0 kb genomic DNA. The same agarose gel was Southern blotted and probed with the P³² labelled 700 bp bgl1 cDNA to identify which genomic restriction fragments shared homology with the bgl1 cDNA. The mapping experiments confirmed that the entire bgl1 gene is contained on the genomic Hind III clone. Pvu II and Bal I restriction fragments which ranged in size from 600 bp to 1500 bp hybridized with the 700 bp DNA bgl1 clone and were chosen for subcloning into pUC218 phagemid. After cloning these fragments into the phagemid, the Pvu II and Bal I subclones were then sequenced using the dideoxy chain termination method of Sanger et al (1977). It was then determined from this sequencing that the overlapping sequences of the subclones aligned with a single contiguous sequence totaling 3033 bp within which the nucleotide sequence was determined on both strands.

### Example 10

### Construction of pSASβ-glu

The starting vector for the construction of pSASß-glu was the plasmid pSAS. pSAS was constructed in the following way. pUC100 (a commercially available plasmid vector) was digested with the restriction enzyme SmaI and the 5' phosphate groups subsequently removed by digestion with calf intestinal alkaline phosphatase. The linear vector fragment was purified from undigested vector and protein by agarose gel electrophoresis followed by isolation of the linear vector DNA from the isolated gel slice by electroelution. The amdS gene was isolated as a 2.4 kb SstI restriction fragment following separation from the vector sequences (contained in - Hynes, M.J., Corrick, C.M., and King, J.A., "Isolation of genomic clones containing the amdS gene of Aspergillus nidulans and their use in the analysis of structural and regulatory mutations", Mol. Cell. Biol., Vol. 3 (1983), pp. 1430-1439). The 2.4 kb SstI amdS fragment and the 2.7 kb pUC100 vector fragment were then ligated together (Sambrook et al., supra) and the ligation mix transformed and propagated in the E. coli host strain, JM101.

pSASß-glu was constructed by digesting pSAS with the restriction enzyme Hind III, and purifying the linear fragment as described above. Into this Hind III treated pSAS vector fragment was ligated a 6.0 kb Hind III fragment of T. reesei genomic DNA that contained all of the coding region of the bgl1 gene along with sequences necessary for the genes transcription and translation.

### Example 11

### Preparation of BGL1 Deletion Vector

The gene replacement vector pUCΔβ-Glu A/R pyr, illustrated in Figure 3B, was constructed by cloning a 6.0 kb genomic HindIII fragment, known to contain the entire bgl1 gene, into the polylinker of pUC218 which had been cut with HindIII and the ends dephosphorylated with calf intestinal alkaline phosphatase. The coding region for the bgl1 gene was then removed from this plasmid by digesting the plasmid with ApaI and EcoRV at unique ApaI and EcoRV restriction sites situated at the very 5' and 3' end of the bgl1 open reading frame and isolating the linear plasmid DNA. The restriction site ends were made blunt with T4 DNA polymerase. This plasmid was then ligated with an isolated 2412 bp Hind III/Bam HI restriction fragment containing the pyrG gene from Aspergillus niger (Hartingsreldt et al., Mol. Gen. Genet. 206:71-75 (1987) in which the restriction ends were made blunt by treatment with T4 DNA polymerase to create pUCΔβGlu A/R pyr (Fig. 3B).

### Example 12

### Isolation of Protoplasts

Mycelium was obtained by inoculating 100 ml of YEG (0.5% yeast extract, 2% glucose) in a 500 ml flask with about 5x10⁷ T. reesei cells. The flask was then incubated at 37°C with shaking for about 16 hours. The mycelium was harvested by centrifugation at 2,750 x g. The harvested mycelium were further washed in 1.2 M sorbitol solution and resuspended in 40 ml of Novozym^{R}, which is the tradename for a multi-component enzyme system containing 1,3-alpha-glucanase, 1,3-beta-glucanase, laminarinase, xylanase, chitinase and protease from Novo Biolabs, Danbury, Ct., solution containing 5 mg/ml Novozym^{R} 234; 5 mg/ml MgSO₄·7H₂O: 0.5 mg/ml bovine serum albumin; 1.2 M sorbitol. The protoplasts were removed from cellular debris by filtration through Miracloth (Calbiochem Corp.) and collected by centrifugation at 2,000 x g. The protoplasts were washed three times in 1.2 M sorbitol and once in 1.2 M sorbitol, 50 mM CaCl₂, centrifuged and resuspended. The protoplasts were finally resuspended at a density of 2 x 10⁸ protoplasts per ml of 1.2 M sorbitol, 50 mM CaCl₂.

### Example 13

### Transformation of Fungal Protoplasts with pSASβ-glu

200 µl of the protoplast suspension prepared in Example 12 was added to 20 µl (20 µg) of pSASβ-glu in TE buffer (10 mM Tris, pH 7.4; 1 mM EDTA) and 50 µl of a polyethylene glycol (PEG) solution containing 25% PEG 4000, 0.6 M KCl and 50 mM CaCl₂. This mixture was incubated on ice for 20 minutes. After this incubation period 2.0 ml of the above-identified PEG solution was added thereto, the solution was further mixed and incubated at room temperature for 5 minutes. After this second incubation, 4.0 ml of a solution containing 1.2 M sorbitol and 50 mM CaCl₂ was added thereto and this solution was further mixed. The protoplast solution was then immediately added to molten aliquot's of Vogels Medium N (3 grams sodium citrate, 5 grams KH₂PO₄, 2 grams NH₄NO₃, 0.2 grams MgSO₄·7H₂O, 0.1 gram CaCl₂·2H₂O, 5 µg α-biotin, 5 mg citric acid, 5 mg ZnSO₄·7H₂O, 1 mg Fe(NH₄)₂·6H₂O, 0.25 mg CuSO₄·5H₂O, 50 µg MnSO₄·4H containing an additional 1% glucose, 1.2 M sorbitol and 1% agarose. The protoplast/medium mixture was then poured onto a solid medium containing the same Vogel's medium as stated above containing in addition acetamide as a nitrogen source. Since T. reesei does not contain a functional equivalent to the amdS gene only transformants will grow on this medium. These colonies were subsequently transferred and purified on a solid Vogel's medium N containing as an additive, 1% glucose. The bgl1 gene inserted transformant strain is called A83pSASßGlu.

Stable transformants can be distinguished from unstable transformants by their faster growth rate and the formation of circular colonies with a smooth rather than ragged outline on solid culture medium. Additionally, in some cases, a further test of stability can be made by growing the transformants on solid non-selective medium, harvesting the spores from this culture medium and determining the percentage of these spores which will subsequently germinate and grow on selective medium.

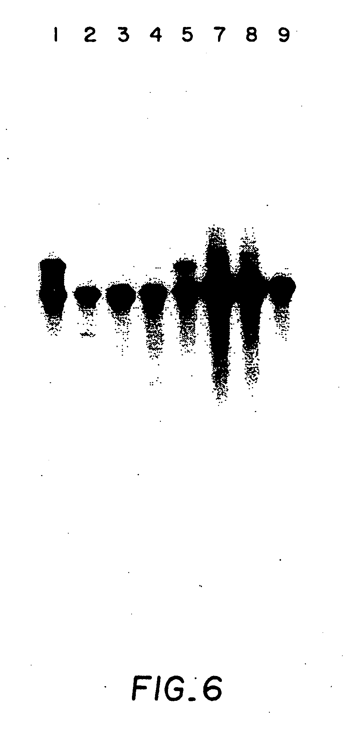

Figure 6 is an autoradiograph of a Southern blot using the P³² labelled 700 bp fragment as a probe, of the different transformants with enhanced copies of the bgl1 gene (lanes 1-8) using genomic T. reesei from an overproducing strain digested with Hind III as a control (lane 9). This autoradiograph clearly shows that the transformants contained enhanced amount of the bgl1 gene compared with the control.

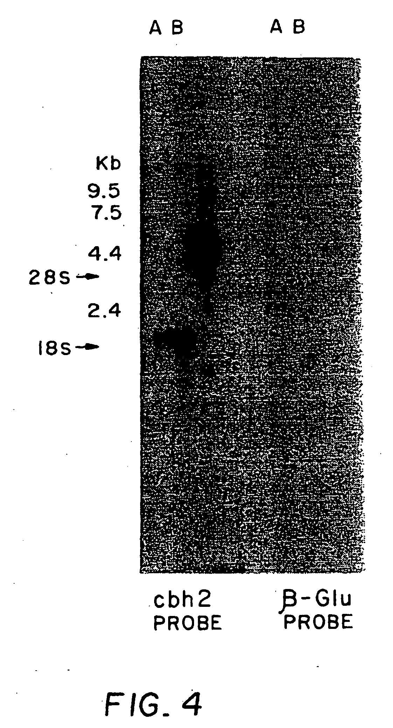

Figure 4 is an autoradiograph of a Northern blot of RNA isolated from one of the transformed strains (lane A) produced by the present invention following induction with soporose illustrating a corresponding increase in the levels of bgl1 message when compared to the parental strain of T. reesei (lane B).

Besides visual analysis of the transformants, quantitative analysis was also completed by cutting the appropriate bands out of the Nytran^{R} membrane and counting the radioactive label present therein in a scintillation counter. This experiment was performed to obtain a more precise estimate of the relative amounts of message as shown in Table III below:

**TABLE III**

| CPM | Parental Trichoderma reesei strain | Transformed Trichoderma reesei strain |
|---|---|---|
| CPM β-glu message | 14.4 | 25.4 |
| CPM CBHII | 227.1 | 95.2 |
| CPM β-glu/CBHII | 0.0634 | 0.2668 |

Table III illustrates that the transformant produced by the process described above has extra β-glucosidase mRNA and hence an increase in β-glucosidase enzyme resulting in an increase in specific activity.

### Example 14

### Transformation of Fungal Protoplasts with pUCΔβGlu A/R pyr4

Mutants of T. reesei lacking the coding sequence for the extracellular β-glucosidase gene, bglI, were obtained by a targeted gene replacement event. pUCΔβGlu A/R pyr4 plasmid was digested with Hind III to obtain a linear HindIII fragment in which the bglI coding sequences were replaced with the pyrG gene from Aspergillus niger. Protoplasts were transformed with the linear DNA fragment containing the bgl1 flanking sequences and the pyr4 by the methods of Examples 12 and 13. The deletion transformants were called Δ12 and Δ36. After transformation, the protoplast solution was then added to molten aliquots of Vogel's Medium N containing an additional 1% glucose, 1.2 M sorbitol and 1% agarose. The protoplast/mdium mixture was then pourred into a solid medium containing the same Vogel's medium N. No uridine was present in the medium and therefore only transformed colonies were able to grow as a result of complementation of the pyr4 mutation of the T. reesei strain RL-P37 by the wild type pyr4 gene inserted in the DNA fragment. Stable transformants were then selected by the method recited in Example 13.

### Example 15

### Analysis of the Transformants

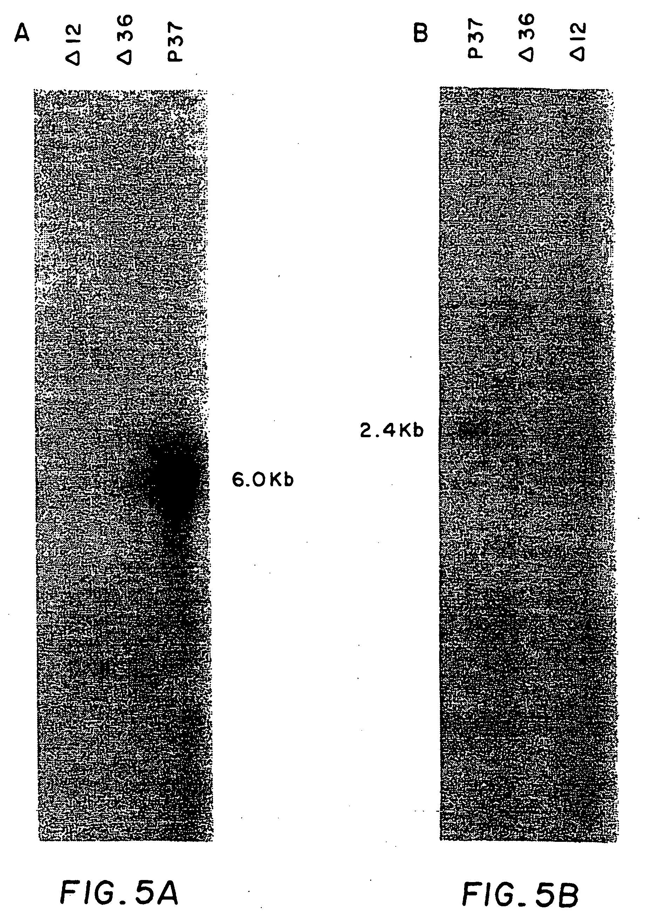

The transformants were analyzed for the presence or absence of the bgl1 gene using the 700 bp cDNA probe recited above. The transformants were digested using HindIII. Total genomic DNA from selected transformants was digested with HindIII restriction enzyme, run on a 1% agarose gel, transferred to Nitran^{R} membrane and probed with a P³² labelled 700 bp cDNA rectied above and visualized by autoradiography on X-ray film. The results of this analysis are set forth in Figure 5A illustrate that the transformants (Δ12 and Δ36) did not contain a band corresponding to the bgl1 gene whereas the wild type strain (RL-P37, i.e., P-37) did.

mRNA isolated from the transformants of Example 14 and analyzed on a Northern blot, as in Example 2. As indicated in Figure 5B, Northern blot analysis using the P³² labelled 2.2 Kb ApaI/EcoRV bgl1 probe indicated that bgl1 specific mRNA was present in T. resei RL-P37 pyrG69 and is absent in the transformants Δ12 and Δ36.

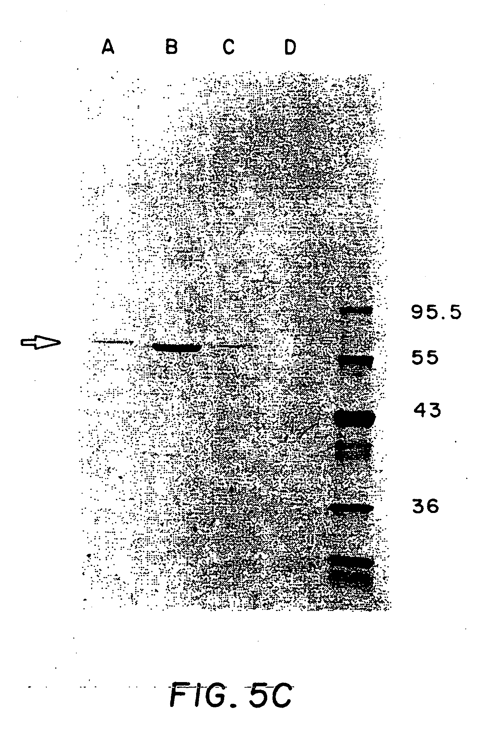

Protein was recovered as per Example 8 above and then analyzed for the presence of β-glucosidase by use of polyclonal antibodies (from rabbits challenged with pure β-glucosidase) tagged with horseradish peroxidase to permit detection. The antibodies were used to identify pure β-glucosidase (100 ng -- Column A; 1000 ng -- Column B); cellulase produced from wild type T. reesei (Column C); and from cellulase produced by a T. reesei strain genetically engineered to delete the β-glucoidase gene (Column D). The results of this analysis are set forth in Figure 5C and show that only Column D did not contain β-glucosidase.

While the invention has been described in terms of various preferred embodiments, the skilled artisan will appreciate that various modifications, substitutions, omissions, and changes may be made without departing from the scope thereof. Accordingly, it is intended that the scope of the present invention be limited solely by the scope of the following claims, including equivalents thereof.

Also disclosed herein are the following:
1. A process for expressing enhanced extracellular β-glucosidase in a filamentous fungus comprising expressing a fungal DNA sequence encoding enhanced β-glucosidase in a recombinant host microorganism, said recombinant host microorganism being a filamentous fungus transformed with an expression vector containing said DNA sequence.
2. The process according to paragraph 1, wherein said expression vector comprises at least one additional copy of a fungal β-glucosidase gene encoding for extracellular β-glucosidase expression.
3. The process according to paragraph 1, further comprising the step of isolating transformants having enhanced β-glucosidase expression.
4. The process according to paragraph 3, further comprising the steps of:
   (a) culturing said transformants under conditions to permit growth of said transformants; and
   (b) isolating a recombinant fungal cellulase composition produced from said transformants.
5. The process according to paragraph 4, further comprising the step of purifying β-glucosidase from said isolated recombinant fungal cellulase composition.
6. The process according to paragraph 1, wherein said filamentous fungus is selected from the genus of Trichoderma, Aspergillus, Neurospora, Humicola and Penicillium.
7. The process according to paragraph 6, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum or Penicillium oxalicum.
8. The process according to paragraph 2, wherein said expression vector comprises all of a coding region of a fungal β-glucosidase gene and sequences necessary for the β-glucosidase gene's transcription and translation.
9. The process according to paragraph 8, wherein said β-glucosidase gene is a bgl1 gene derived from Trichoderma reesei.
10. The process according to paragraph 9, wherein said bgl1 gene substantially comprises the following amino acid and nucleotide sequence from 311 to 2679 of Figure 1.
11. The process according to paragraph 4, wherein said recombinant fungal cellulase composition is isolated by:
   (a) centrifuging said culture medium containing said transformants having enhanced β-glucosidase activity to form a supernatant and a pellet; and
   (b) filtering said supernatant to obtain a recombinant fungal cellulase composition.
12. The process according to paragraph 11, wherein an antimicrobial agent is added to said recombinant fungal cellulase composition after filtration.
13. Transformants having enhanced extracellular β-glucosidase expression produced by the process according to paragraph 3.
14. A fungal cellulase composition derived from a filamentous fungus which is enriched by recombinant means in β-glucosidase.
15. The fungal cellulase composition according to paragraph 14, wherein said filamentous fungus is selected from the genus of Trichoderma, Aspergillus, Neurospora, Humicola, and Penicillium.
16. The fungal cellulase composition according to paragraph 14, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum, Penicillium oxalicum, or admixtures thereof.
17. A process for expressing cellulases but does not express extracellular β-glucosidase in a filamentous fungus comprising expressing a fungal DNA sequence encoding cellulases but not encoding β-glucosidase in a recombinant host microorganism, said recombinant host microorganism being a filamentous fungus transformed with an expression vector containing said DNA sequence.
18. The process according to paragraph 17, wherein said expression vector comprises genes encoding for cellulase expression but does not contain a β-glucosidase gene encoding for β-glucosidase expression.
19. The process according to paragraph 17, further comprising the step of isolating transformants which are able to express other cellulases but do not express β-glucosidase.
20. The process according to paragraph 19, further comprising the steps of:
   (a) culturing said transformants under conditions to permit growth of said transformants; and
   (b) isolating a recombinant fungal cellulase composition produced from said transformants.
21. The process according to paragraph 20, further comprising the step of purifying cellulases from said isolated recombinant fungal cellulase composition.
22. The process according to paragraph 17, wherein said filamentous fungus is selected from the genus of Trichoderma, Aspergillus, Neurospora, Humicola, and Penicillium.
23. The process according to paragraph 22, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum or Penicillium oxalicum.
24. The process according to paragraph 18, wherein said deleted β-glucosidase gene is a bgl1 gene derived from Trichoderma reesei.
25. The process according to paragraph 21, wherein said deleted bgl1 gene substantially comprises the following amino acid and nucleotide sequence from base numbers 311 to 2679 of Figure 1.
26. The process according to paragraph 20, wherein said recombinant fungal cellulase composition is isolated by:
   (a) centrifuging said culture medium containing said transformants containing cellulase expression but do not express β-glucosidase to form a supernatant and a pellet; and
   (b) filtering said supernatant to obtain a recombinant fungal cellulase composition.
27. The process according to paragraph 26, wherein an antimicrobial agent is added to said recombinant fungal cellulase composition after filtration.
28. Transformants which express cellulases but do not express β-glucosidase produced by the process according to paragraph 19.
29. A fungal cellulase composition derived from a filamentous fungus which comprises by recombinant means all cellulases except β-glucosidase.
30. The fungal cellulase composition according to paragraph 29, wherein said filamentous fungus is selected from the genus of Trichoderma, Aspergillus, Neurospora, Humicola, and Penicillium.
31. The fungal cellulase composition according to paragraph 30, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum, Penicillium oxalicum, or admixtures thereof.
32. A process for expressing an altered extracellular β-glucosidase in a filamentous fungus comprising expressing a fungal DNA sequence encoding an altered β-glucosidase in a recombinant host microorganism, said recombinant host microorganism being a filamentous fungus transformed with an expression vector containing said DNA sequence.
33. The process according to paragraph 32, wherein said expression vector comprises an altered fungal β-glucosidase gene encoding for altered extracellular β-glucosidase.
34. The process according to paragraph 32, further comprising the step of isolating transformants having expression of altered β-glucosidase.
35. The process according to paragraph 34, further comprising the steps of:
   (a) culturing transformants under conditions to permit growth of said transformants; and
   (b) isolating a recombinant fungal cellulase composition produced from said transformants.
36. The process according to paragraph 35, further comprising the step of purifying said altered fungal β-glucosidase from said isolated recombinant fungal cellulase composition.
37. The process according to paragraph 32, wherein said filamentous fungus is selected from the genus of Trichoderma, Aspergillus, Neurospora, Humicola, and Penicillium.
38. The process according to paragraph 37, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum or Penicillium oxalicum.
39. The process according to paragraph 32, wherein said altered fungal β-glucosidase gene is a bgl1 gene derived from Trichoderma reesei.
40. The process according to paragraph 39, wherein the bgl1 gene amino acid and nucleotide sequence of Figure 1 can be altered.
41. The process according to paragraph 34, wherein said recombinant fungal cellulase composition is isolated by:
   (a) centrifuging said culture medium containing transformants having said altered β-glucosidase expression to form a supernatant and a pellet; and
   (b) filtering said supernatant to obtain a recombinant fungal cellulase composition.
42. The process according to paragraph 41, wherein an antimicrobial agent is added to said recombinant fungal cellulase composition after filtration.
43. Transformants expressing said altered fungal β-glucosidase produced by the process according to paragraph 34.
44. A fungal cellulase composition derived from a filamentous fungus which is an altered β-glucosidase produced by recombinant means.
45. The fungal cellulase composition according to paragraph 44, wherein said filamentous fungus is selected from the genus of Trichoderma, Aspergillus, Neurospora, Humicola, and Penicillium.
46. The fungal cellulase composition according to paragraph 45, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum, Penicillium oxalicum, or admixtures thereof.
47. A method for enhancing the degradation of cellulose to glucose by using a recombinant fungal cellulase composition derived from a filamentous fungus which has enhanced β-glucosidase activity.
48. The method of use according to paragraph 47, wherein said filamentous fungus is selected from the genus of Trichoderma, Aspergillus, Neurospora, Humicola, and Penicillium.
49. The method of use according to paragraph 48, wherein said filamentous fungus is Trichoderma reesei, Trichoderma viridae, Trichoderma koningii, Aspergillus niger, Aspergillus nidulans, Aspergillus wentii, Aspergillus oryzae, Aspergillus phoenicis, Neurospora crassa, Humicola grisea, Penicillium pinophilum, Penicillium oxalicum, or admixtures thereof.
50. A composition that increases the rate and extent of production of glucose from cellulosics or hetero-glycans, said composition comprising a recombinant fungal cellulase composition having enhanced β-glucosidase activity.
51. The composition according to paragraph 50, wherein said recombinant fungal cellulase composition having enhanced β-glucosidase activity may be used to degrade cellulosics present in feedstock, biomass and sludge.
52. A method of using the composition of paragraph 50, said method comprising adding said recombinant fungal cellulase composition to yeast and biomass to form a mixture and culturing said mixture at a sufficient temperature and for a sufficient period of time to produce ethanol.
53. A method of using the transformants of paragraph 13, said method comprising adding said transformants to yeast and biomass to form a mixture and culturing said mixture at a sufficient temperature and for a sufficient period of time to produce ethanol.
54. The method according to paragraphs 52 or 53, wherein said yeast is B. clausenii, S. cerevisiae, Celluloyticus acidothermophilium, C. brassicae, C. lustinaniae, S. uvarum or Schizosaccharomyces pombe.
55. A fungal cellulase composition containing enhanced extracellular fungal β-glucosidase activity comprising at least one additional extracellular β-glucosidase wherein said extracellular β-glucosidase substantially comprises the amino acid and nucleotide sequence from base numbers 311 to 2679 of Figure 1.
56. A method of using purified β-glucosidase produced from the process according to paragraph 5, in food additives to enhance the flavor and aroma of foods.
57. A method of using the recombinant fungal cellulase composition produced according to paragraph 14, in food additives to enhance the flavor and aroma.
58. A detergent composition comprising a cleaning effective amount of a surfactant and at least .0001 weight percent of a recombinant fungal cellulase composition according to paragraph 14.
59. A detergent composition comprising a cleaning effective amount of a surfactant and at least .0001 weight percent of a recombinant fungal cellulase composition according to paragraph 29.
60. A detergent composition comprising a cleaning effective amount of a surfactant and at least .0001 weight percent of a recombinant fungal cellulase composition according to paragraph 44.
61. A nucleotide sequence of a bgl1 gene which entire sequence or a portion can be labeled and used as a probe wherein the bgl1 gene has the nucleotide sequence of Figure 1.
62. A method of using the probe according to paragraph 61, for identifying a β-glucosidase gene from a filamentous fungus.
63. The method according to paragraph 62, wherein said probe is derived from Trichoderma reesei.
64. The method according to paragraph 62, wherein one or more probes can be used to identify said β-glucosidase gene from a filamentous fungus.
65. A method for producing cellooligosaccharides which comprises contacting cellulose containing materials with a cellulase composition free of β-glucosidase at a pH of from about 4.5 to about 8.

## Claims

1. A process for preparing a strain filamentous fungi which produces cellulase compositions deficient in extracellular β-glucosidase,
the process comprising use of a cloned fungal extracellular β-glucosidase gene for deletion or disruption of the filamentous fungi genome,
whereby an extracellular β-glucosidase gene is either
(i) deleted from the genome and therefore cannot be expressed by the recombinant host microorganism; or
(ii) disrupted in the genome so that a functional extracellular β-glucosidase cannot be produced by the recombinant host microorganism,
and wherein the filamentous fungus is Trichoderma reesei or said cloned extracellular β-glucosidase gene is a bgl1 gene from Trichoderma reesei.

2. The process according to claim 1 wherein the composition is free of extracellular β-glucosidase.

3. The process according to claim 1 wherein the deleted or disrupted β-glucosidase gene is a bgl1 gene.

4. The process according to claim 1 wherein the filamentous fungi is Trichoderma reesei.

5. The process according to claim 4 wherein the strain of Trichoderma reesei is RLP37 pyrG69.

6. The process according to claim 4 wherein the bgl1 gene is deleted.

7. The process according to claim 6 wherein the bgl1 gene is totally deleted and replaced with another known gene.

8. The process according to claim 7 wherein the replacing gene is homologous to the filamentous fungi so that the resulting recombinant microorganism does not express any heterologous protein.

9. The process according to claim 7 wherein the replacing gene is the pyrG gene from Aspergillus niger.

10. A process for producing cellulase composition free of extracellular β-glucosidase by culturing a filamentous fungi wherein an extracellular β-glucosidase gene has been either deleted or disrupted by the process of claim 1.

11. The process according to claim 10 wherein the extracellular β-glucosidase gene has been deleted.

12. The process according to claim 11 comprising use of a plasmid encoding an entire bgl1 gene from which the coding region for the bgl1 gene is then removed.

13. The process according to claim 12, wherein said plasmid is an expression vector which comprises genes encoding for cellulase expression but does not contain an extracellular β-glucosidase gene coding for extracellular β-glucosidase expression.

14. The process according to claim 13, further comprising the step of isolating transformants which are able to express other cellulases but which do not express extracellular β-glucosidase.

15. The process according to claim 14, further comprising the steps of:
(a) culturing said transformants under conditions to permit growth of said transformants; and
(b) isolating a recombinant fungal cellulase composition produced from said transformants.

16. The process according to claim 15 further comprising the step of purifying cellulases from said isolated recombinant fungal cellulase composition.

17. The process according to any one of claims 11 to 16, wherein said deleted extracellular β-glucosidase gene is a bgl1 gene and the filamentous fungus is Trichoderma reesei.

18. The process according to claim 17, wherein said bgl1 gene comprises the amino acids encoded by the nucleotide sequence from 311 to 2679 of Figure 1.

19. The process according to claim 15, wherein said recombinant fungal cellulase composition is isolated by:
(a) centrifuging said culture medium containing said transformants to form a supernatant and a pellet; and
(b) filtering said supernatant to obtain a recombinant fungal cellulase composition.

20. The process according to claim 19, wherein an antimicrobial agent is added to said recombinant fungal cellulase composition after filtration.

21. An isolated transformed filamentous fungus which produces cellulases compositions free of extracellular β-glucosidase,
whereby an extracellular β-glucosidase gene in said filamentous fungi genome is either:
(i) deleted from the genome and therefore cannot be expressed by the recombinant host microorganism; or
(ii) disrupted in the genome so that a functional extracellular enzyme β-glucosidase cannot be produced by the recombinant host microorganism,
and wherein the filamentous fungus is Trichoderma reesei or said extracellular β-glucosidase gene is a bgl1 gene from Trichoderma reesei.

## Patentansprüche

1. Verfahren zur Herstellung eines filamentösen Pilzstamms, der exhazelluläre β-Glucosidase defiziente Cellulasezusammensetzungen erzeugt,
wobei das Verfahren die Verwendung eines klonierten, pilzlichen extrazellulären β-Glucosidase-Gens zur Deletion oder Disruption des filamentösen Pilzgenoms umfasst,
wodurch ein extrazelluläres β-Glucosidase-Gen entweder
(i) aus dem Genom deletiert wird und somit durch den rekombinanten Wirtsmikroorganismus nicht exprimiert werden kann; oder
(ii) in dem Genom disruptiert wird, so dass durch den rekombinanten Wirtsmikroorganismus keine funktionelle extrazelluläre β-Glucosidase erzeugt werden kann, und
wobei der filamentöse Pilz Trichoderma reesei oder das klonierte extrazelluläre β-Glucosidase-Gen ein bgl1-Gen von Trichoderma reesei ist.

2. Verfahren nach Anspruch 1, wobei die Zusammensetzung frei ist von extrazellulärer β-Glucosidase.

3. Verfahren nach Anspruch 1, wobei das deletierte oder disruptierte β-Glucosidase-Gen ein bgl1-Gen ist.

4. Verfahren nach Anspruch 1, wobei der filamentöse Pilz Trichoderma reesei ist.

5. Verfahren nach Anspruch 4, wobei der Trichoderma reesei-Stamm RLP37 pyrG69 ist.

6. Verfahren nach Anspruch 4, wobei das bgl1-Gen deletiert wird.

7. Verfahren nach Anspruch 6, wobei das bgl1-Gen vollständig deletiert und durch ein anderes bekanntes Gen ersetzt wird.

8. Verfahren nach Anspruch 7, wobei das Ersatz-Gen homolog zu dem filamentösen Pilz ist, so dass der resultierende rekombinante Mikroorganismus keinerlei heterologes Protein exprimiert.

9. Verfahren nach Anspruch 7, wobei das Ersatz-Gen das pyrG-Gen von Aspergillus niger ist.

10. Verfahren zur Herstellung einer Cellulasezusammensetzung, die frei ist von extrazellulärer β-Glucosidase, durch Kultivieren eines filamentösen Pilzes, wobei ein extrazelluläres β-Glucosidase-Gen durch das VerFahren nach Anspruch 1 entweder deletiert oder disruptiert worden ist.

11. Verfahren nach Anspruch 10, wobei das extrazelluläre β-Glucosidase-Gen deletiert worden ist.

12. Verfahren nach Anspruch 11, umfassend die Verwendung eines Plasmids, welches das gesamte bgl1-Gen codiert, von dem die codierende Region für das bgl1-Gen dann entfernt wird.

13. Verfahren nach Anspruch 12, wobei das Plasmid ein Expressionsvektor ist, der Gene umfasst, die für eine Cellulase-Expression codieren, jedoch kein extrazelluläres β-Glucosidase-Gen enthält, das für eine extrazelluläre β-Glucosidase-Expression codiert.

14. Verfahren nach Anspruch 13, ferner umfassend den Schritt des Isolierens von Transformanten, welche andere Cellulasen exprimieren können, jedoch keine extrazelluläre β-Glucosidase exprimieren.

15. Verfahren nach Anspruch 14, ferner umfassend die Schritte:
(a) Kultivieren der Transformanten unter Bedingungen, welche das Wachstum der Transformanten ermöglichen; und
(b) Isolieren einer durch die Transformanten erzeugten rekombinanten pilzlichen Cellulasezusammensetzung.

16. Verfahren nach Anspruch 15, ferner umfassend den Schritt des Reinigens von Cellulasen aus der isolierten, rekombinanten, pilzlichen Cellulasezusammensetzung.

17. Verfahren nach einem der Ansprüche 11 bis 16, wobei das deletierte extrazelluläre β-Glucosidase-Gen ein bgl1-Gen ist und der filamentöse Pilz Trichoderma reesei ist.

18. Verfahren nach Anspruch 17, wobei das bgl1-Gen die Aminosäuren umfasst, die durch die Nucleotidsequenz von 311 bis 2679 der Figur 1 codiert sind.

19. Verfahren nach Anspruch 15, wobei die rekombinante pilzliche Cellulasezusammensetzung isoliert wird durch:
(a) Zentrifugieren des Kulturmediums, das die Transformanten enthält, um einen Überstand und ein Pellet zu bilden; und
(b) Filtern des Überstands, um eine rekombinante pilzliche Cellulasezusammensetzung zu erhalten.

20. Verfahren nach Anspruch 19, wobei nach der Filtrierung ein antimikrobielles Mittel zu der rekombinanten pilzlichen Cellulasezusammensetzung gegeben wird.

21. Isolierter transformierter filamentöser Pilz, der Cellulasezusammensetzungen erzeugt, die frei von extrazellulärer β-Glucosidase sind,
wobei in dem filamentösen Pilzgenom ein extrazelluläres β-Glucosidase-Gen entweder:
(i) aus dem Genom deletiert ist und somit durch den rekombinanten Wirtsmikroorganismus nicht exprimiert werden kann; oder
(ii) in dem Genom disruptiert ist, so dass durch den rekombinanten Wirtsmikroorganismus kein funktionelles extrazelluläres Enzym β-Glucosidase erzeugt werden kann,
und wobei der filamentöse Pilz Trichoderma reesei oder das extrazelluläre β-Glucosidase-Gen ein bgl1-Gen von Trichoderma reesei ist.

## Revendications

1. Procédé de préparation d'une souche de champignon filamenteux qui produit des compositions de cellulases déficientes en β-glucosidase extracellulaire,
le procédé comprenant l'utilisation d'un gène de β-glucosidase extracellulaire fongique cloné pour la délétion ou la rupture du génome du champignon filamenteux,
par quoi un gène de β-glucosidase extracellulaire est soit :
(i) délété du génome et par conséquent ne peut être exprimé par le micro-organisme hôte recombinant ; soit
(ii)rompu dans le génome de telle sorte que le micro-organisme hôte recombinant ne peut produire une β-glucosidase extracellulaire fonctionnelle,
et dans lequel le champignon filamenteux est *Trichoderma reesei* ou ledit gène de β-glucosidase extracellulaire cloné est un gène *bgl*1 dérivé de *Trichoderma reesei.*

2. Procédé selon la revendication 1 dans lequel la composition est dépourvue de β-glucosidase extracellulaire.

3. Procédé selon la revendication 1 dans lequel le gène de β-glucosidase délété ou rompu est un gène *bgl*1.

4. Procédé selon la revendication 1 dans lequel le champignon filamenteux est *Trichoderma reesei.*

5. Procédé selon la revendication 4 dans lequel la souche de *Trichoderma reesei* est la souche RLP37 pyrG69.

6. Procédé selon la revendication 4 dans lequel le gène *bgl*1 est délété.

7. Procédé selon la revendication 6 dans lequel le gène *bgl*1 est totalement délété ou remplacé par un autre gène connu.

8. Procédé selon la revendication 7 dans lequel le gène remplaçant est homologue du champignon filamenteux de sorte que le micro-organisme recombinant résultant n'exprime aucune protéine hétérologue.

9. Procédé selon la revendication 7 dans lequel le gène de remplacement est le gène pyrU de *Aspergillus niger.*

10. Procédé de production d'une composition de cellulases dépourvue de β-glucosidase extracellulaire par mise en culture de champignons filamenteux dans lesquels un gène de β-glucosidase extracellulaire a été soit délété, soit rompu selon le procédé de la revendication 1.

11. Procédé selon la revendication 10 dans lequel le gène de β-glucosidase extracellulaire a été délété.

12. Procédé selon la revendication 11 comprenant l'utilisation d'un plasmide codant pour l'intégralité du gène *bgl*1, plasmide dont la région qui code pour le gène *bgl*1 est ensuite éliminée.

13. Procédé selon la revendication 12 dans lequel ledit plasmide est un vecteur d'expression qui comprend des gènes codant pour l'expression de cellulases mais ne contient pas de gène de β-glucosidase extracellulaire codant pour l'expression d'une β-glucosidase extracellulaire.

14. Procédé selon la revendication 13, comprenant en outre l'étape consistant à isoler des transformants qui sont capables d'exprimer d'autres cellulases mais qui n'expriment pas de β-glucosidase extracellulaire.

15. Procédé selon la revendication 14, comprenant en outre les étapes consistant à :
(a) mettre en culture lesdits transformants dans des conditions qui permettent la croissance desdits transformants ; et
(b) isoler une composition de cellulases fongiques recombinantes produite par lesdits transformants.

16. Procédé selon la revendication 15 comprenant en outre l'étape consistant à purifier les cellules à partir de ladite composition de cellulases fongiques recombinantes.

17. Procédé selon l'une quelconque des revendications 11 à 16, dans lequel ledit gène de β-glucosidase extracellulaire délété est un gène *bgl*1 et le champignon filamenteux est *Trichoderma reesei.*

18. Procédé selon la revendication 17, dans lequel ledit gène *bgl*1 comprend les acides aminés codés par la séquence de nucléotides 311 à 2679 de la figure 1.

19. Procédé selon la revendication 15, dans lequel ladite composition de cellulases fongiques recombinantes est isolée par :
a) centrifugation dudit milieu de culture contenant lesdits transformants de manière à former un surnageant et un culot ; et
b) filtration dudit surnageant de manière à obtenir une composition de cellulases fongiques recombinantes.

20. Procédé selon la revendication 19, dans lequel un agent antimicrobien est ajouté à ladite composition de cellulases fongiques recombinantes après la filtration.

21. Champignon filamenteux transformé isolé qui produit des compositions de cellulases dépourvues de β-glucosidase extracellulaire,
par quoi un gène de β-glucosidase extracellulaire dans ledit génome du champignon filamenteux est soit :
(i) délété du génome et ne peut donc pas être exprimé par le micro-organisme hôte recombinant ; soit
(ii) rompu dans le génome de sorte que le micro-organisme hôte recombinant ne peut pas produire une enzyme β-glucosidase extracellulaire fonctionnelle,
et dans lequel le champignon filamenteux est *Trichoderma reesei* ou ledit gène de β-glucosidase extracellulaire est un gène *bgl*1 de *Trichoderma reesei.*
